# EUROPEAN PATENT APPLICATION

(11) **EP 4 124 623 A1**
(43) Date of publication of application: **01.02.2023**
(21) Application number: 21382692.8
(22) Date of filing: 27.07.2021
(51) Int. Cl.: C07K 16/28, A61K 39/00, A61P 21/00

(54) **CD36 INHIBITOR FOR USE IN PROMOTING MUSCLE REGENERATION**

(71) Applicant: Universitat Pompeu Fabra, 08002 Barcelona (ES); Fundació Institut de Recerca Biomèdica (IRB Barcelona), 08028 Barcelona (ES); Institució Catalana de Recerca I Estudis Avançats, 08010 Barcelona (ES)
(72) Inventor: MUÑOZ CÁNOVES, Purificación, 08002 Barcelona (ES); PERDIGUERO SANTAMARIA, Eusebio, 08002 Barcelona (ES); SERRANO SÁNCHEZ, Antonio Luis, 08002 Barcelona (ES); MOISEEVA, Victoria, 08002 Barcelona (ES); RAYA CHAMORRO, Marina, 08002 Barcelona (ES); AZNAR BENITAH, Salvador, 08028 Barcelona (ES)
(74) Representative: Hoffmann Eitle

(57) **Abstract**

The present invention refers to the use of CD36 inhibitors for promoting muscle regeneration, or for the treatment of muscle degeneration.

## Description

### FIELD OF THE INVENTION

The present invention refers to the medical field. Particularly, the present invention refers to the use of CD36 inhibitors for promoting muscle regeneration, or for the treatment of muscle degeneration.

### STATE OF THE ART

Tissue regeneration is necessary for life. Successful tissue regeneration requires coordinated communication between resident stem cells and surrounding niche cells. Examples can be found in the crosstalk between skeletal muscle stem cells (called satellite cells, SCs) and macrophages (Mϕs) or fibroadipogenic progenitors (FAPs; also called mesenchymal cells), between hematopoietic stem cells and osteoblastic or nerve-adrenergic cells, and between epidermal stem cells and dermal fibroblasts or adipogenic cells. However, the identities of the niche cellular components and secreted factors that regulate tissue regenerative responses are not fully characterized.

During aging, tissue regenerative functions decline, in part due to stem cell-intrinsic accumulation of damage (e.g., DNA damage, loss of proteostasis, and oxidative damage), the functional decay of niche cells, and an age-related increase of inflammation (inflammaging) by as-yet uncharacterized mechanisms.

Cellular senescence is a state of durable cell cycle arrest of dysfunctional cells, which acquire a bioactive senescence-associated secretory phenotype (SASP). Senescent cells accumulate with aging and age-associated diseases, limiting life- and healthspan in mice. In contrast, senescent cells have documented beneficial effects as tumor suppressors and during embryo development, liver repair and skin wound healing or reprogramming. However, little is known about the molecular identity of senescent cells *in vivo* or their contribution to tissue regeneration.

There is an unmet medical need of finding reliable strategies for promoting tissue regeneration, particularly muscle regeneration. The present invention is focused on solving this problem and a new strategy is herein provided aimed at promoting muscle regeneration and/or treating muscle degeneration, particularly in the context of age-associated diseases or of muscular dystrophy.

### DESCRIPTION OF THE INVENTION

### Brief description of the invention

The inventors of the present invention have identified senescent cells as integral components of the skeletal muscle regenerative niche that repress regeneration at all stages of life. Using a new sorting protocol, the inventors isolated different senescent cell types from damaged muscles of young and geriatric mice. Transcriptome, chromatin, and pathway analyses revealed conservation of two universal senescence hallmarks (inflammation and fibrosis) across cell type, regeneration time and aging, while cell identity traits were preserved. Senescent cells create an "aged-like" inflamed niche, mirroring inflammation-associated with aging (inflammaging), that arrests stem cell proliferation and regeneration.

Such as it is shown in the results provided below, the reduction of senescent cell inflammatory secretome, through CD36 inhibition or neutralization, accelerates regeneration in young and geriatric mice. Conversely, transplantation of senescent cells delays regeneration. Thus, these results define a senescence blueprint for muscle and uncover unproductive interactions between senescent cells and stem cells in regenerative niches, which can be overcome. Since senescent cells also accumulate in human muscles, our findings open potential avenues toward improved muscle repair throughout life.

These results indicate that CD36 blockade acts as a senomorphic rather than a senolytic, principally affecting the inflammatory SASPs. Notably, CD36 blockade improves muscle regeneration in both young and old mice while reducing inflammation and fibrosis. Furthermore, regenerating muscles from anti-CD36 antibody-treated old mice showed increased force. Improved muscle regeneration after CD36 neutralization was comparable to the improvement observed after senescent cell elimination (either pharmacological or genetic). These results thus indicate that CD36 inactivation unleashes repressive inflammatory downstream effects in SCs within the regenerative niche. This is consistent with myogenesis repression upon inhibition of CCR2 signalling in SCs by the chemokines CCL2, CCL7, and CCL8.

The authors also silenced CD36 in freshly sorted senescent cells using specific siRNA (si-Cd36). Scrambled siRNA (si-Scramble) was used as a control. Upon transplantation to injured muscle, si-Scramble-Sen cells increased the number of senescent cells in the damaged area and delayed its regeneration, whereas engraftment of Cd36- silenced senescent cells had no negative effects.

Consistent with this result, the SASPs produced by freshly sorted senescent cells reduced SC proliferation in *ex vivo* transwell assays, but this effect was not observed when Cd36 was silenced in senescent cells before co-culturing with SCs.

Overall, these results demonstrate that CD36 is crucial for the paracrine effects of senescent cells on muscle regeneration by regulating the production of SASPs (principally via pro-inflammatory factors) *in vivo.*

In summary, the authors of the present invention have demonstrated that CD36 neutralization reduces the inflammatory secretome of the senescent cells, thus contributing to accelerate muscle regeneration in young and geriatric mice.

So, the first embodiment of the present invention refers to CD36 inhibitors for use in promoting muscle regeneration and/or in the treatment of muscle degeneration.

The second embodiment of the present invention refers to a pharmaceutical composition comprising CD36 inhibitors and, optionally, pharmaceutically acceptable carriers or excipients, for use in promoting muscle regeneration and/or in the treatment of muscle degeneration.

Alternatively, the first and second embodiments of the present invention refer to a method for promoting muscle regeneration and/or for treating of muscle degeneration, which comprises the administration of a therapeutically effective dose of CD36 inhibitors, or of a pharmaceutical composition comprising CD36 inhibitors.

In a preferred embodiment, the CD36 inhibitor is an anti-CD36 antibody, or any antigen-binding fragment thereof comprising: a single chain antibody, F(ab')2, Fab, scFab or scFv.

In a preferred embodiment the anti-CD36 antibody is a humanized antibody or a human antibody.

In a preferred embodiment the CD36 inhibitor is a siRNA, a shRNA, an iRNA, an antisense RNA or DNA, specifically targeting CD36.

In a preferred embodiment, the CD36 inhibitors are used for treating age-associated diseases or of muscular dystrophy.

The third embodiment of the present invention refers to an *in vitro* method for screening candidate molecules for promoting muscle regeneration and/or for treating muscle degeneration, which comprises: a) assessing the level of expression of the CD36 after administering a candidate molecule and b) wherein, if after administering the candidate molecule, a statistically significant inhibition of CD36 expression is observed, with respect to a pre-established threshold expression level, this is indicative that the candidate molecule is effective in promoting muscle regeneration, or in the treatment of muscle degeneration.

In a preferred embodiment, the *in vitro* method for screening candidate molecules is focused on finding effective therapies for the treatment of age-associated diseases and/or muscular dystrophy.

The fourth embodiment of the present invention refers to an *in vitro* method for monitoring the response of a patient to a treatment for promoting muscle regeneration and/or for treating muscle degeneration, which comprises: a) assessing the level of expression of the CD36 after administering treatment and b) wherein, if after administering the treatment, a statistically significant inhibition of CD36 expression is observed, with respect to a pre-established threshold expression level, this is indicative that the patient is responding to the treatment.

In a preferred embodiment, the *in vitro* method for monitoring the response of a patient to a treatment for promoting muscle regeneration and/or for treating muscle degeneration is focused on monitoring the response of a patient to a treatment of age-associated diseases and/or muscular dystrophy.

The fifth embodiment of the present invention refers to the *in vitro* use of CD36 for screening candidate molecules able to promote muscle regeneration and/or treat muscle degeneration, particularly for screening and/or producing candidate molecules for treating age-associated diseases and/or muscular dystrophy.

The sixth embodiment of the present invention refers to the *in vitro* use of CD36 for monitoring the response of a patient to a treatment for improving muscle regeneration and/or for treating muscle degeneration, particularly for monitoring the response of a patient to a treatment against age-associated diseases and/or muscular dystrophy.

For the purpose of the present invention the following terms are defined:
- The term "CD36 inhibitor" refers to any compound or molecule reducing or inhibiting the activity and/or expression of CD36. This term includes any compound which specifically targets the mRNA and inhibits mRNA translation, or even antagonists selective for CD36. As explained above, the inhibitor is preferably anti-CD36 antibody, or any antigen-binding fragment thereof comprising: a single chain antibody, F(ab')2, Fab, scFab or scFv. The inhibitor can also be a siRNA, a shRNA, an iRNA, an antisense RNA or DNA, specifically targeting CD36. Moreover, according to the present invention, the expression "CD36 inhibitor" also includes any chemical compound or small molecule able to inhibit, partially or totally, the activity and/or expression of CD36.
- The term "comprising" means including, but not limited to, whatever follows the word "comprising". Thus, the use of the term "comprising" indicates that the listed elements are required or mandatory, but that other elements are optional and may or may not be present.
- By "consisting of' it is meant including, and limited to, whatever follows the expression "consisting of'. Thus, the expression "consisting of' indicates that the listed elements are required or mandatory, and that no other elements may be present.
- "Pharmaceutically acceptable excipient or carrier" refers to a compound that may optionally be included in the composition of the invention and that causes no significant adverse toxicological effects to the patient.
- By "therapeutically effective dose" of a composition of the invention is intended an amount that, when administered as described herein, brings about a positive therapeutic response in the patient. The exact amount required will vary from subject to subject, depending on the age, the general condition of the subject, the severity of the condition being treated, the mode of administration, etc. An appropriate "effective" amount in any individual case may be determined by one of ordinary skill in the art using routine experimentation, based upon the information provided herein.
- "Fab" are antibody fragments with a size of around 50 KDa are the antigen-binding domains of an antibody molecule, containing one constant and one variable domain of each of the heavy and the light chains. The fragments which contain disulfide bridge thiols are called "Fab' fragments", whereas those lacking the thiol functional group are termed "Fab fragments". To produce "Fab" fragments, two different methods can be employed. The primary method is via enzymatic/chemical cleavage of the whole antibody, in which the whole antibody is cleaved by enzyme (such as papain, pepsin, and ficin) to form "F(ab')2" fragments, followed by the reduction of those fragments to yield "Fab" fragments. An alternative method is through the recombinant synthesis of "F(ab')2" antibody fragments, followed by chemical reduction of these fragments to yield Fab units. A "F(ab')2" fragment, which retains a small part of the Fc hinge region, has two antigen binding regions that may increase the affinity to antigen. Reduction of "F(ab')2" fragments produces two monovalent Fab' fragments, which have a free sulfhydryl group that is useful for conjugation with other molecules. Although the utilization of enzymatic/chemical cleavage methods to generate "Fab" fragments is convenient and efficient, it requires a large quantity of monoclonal antibody as starting material. A single chain "Fab" fragment (scFab) can lead to improved function and production of "Fab" fragments. According to some studies, "scFab" fragments show superior antigen-binding ability compared to "Fab" and compensate for some of the disadvantages of the soluble Fab production in E. coli.
- The term "single-chain variable fragment" or "scFv" refers to a fusion protein comprising the variable domains of the heavy chain (VH) and light chain (VL) of an antibody linked to one another with a peptide linker. The term also includes a disulfide stabilized Fv (dsFv). Methods of stabilizing scFvs with disulfide bonds are disclosed in Reiter et al., 1996. Nat Biotechnol. 14(10):1239-45.

### Description of the figures

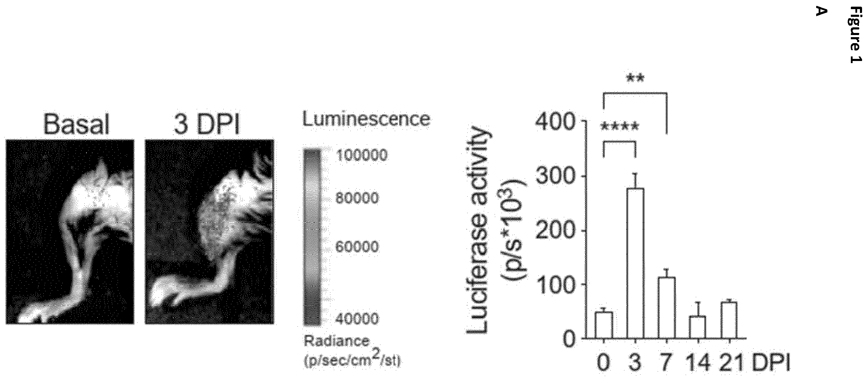

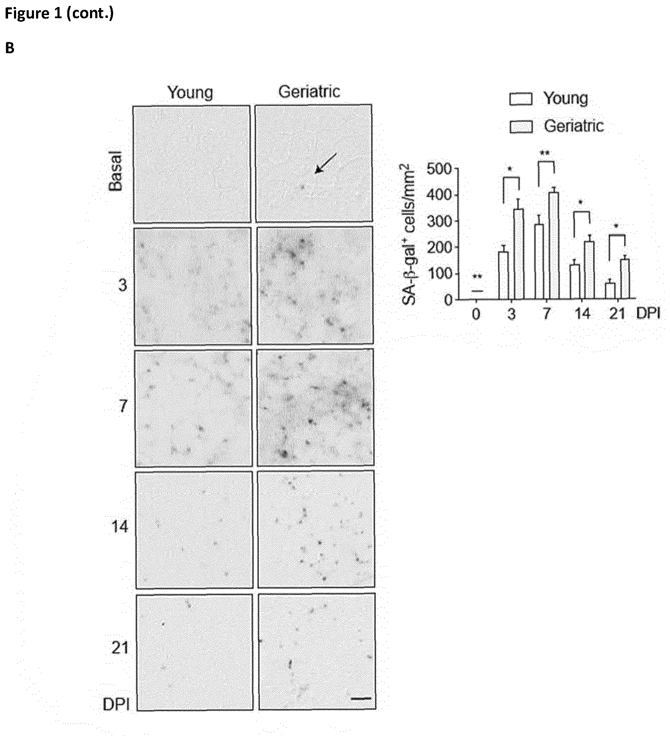

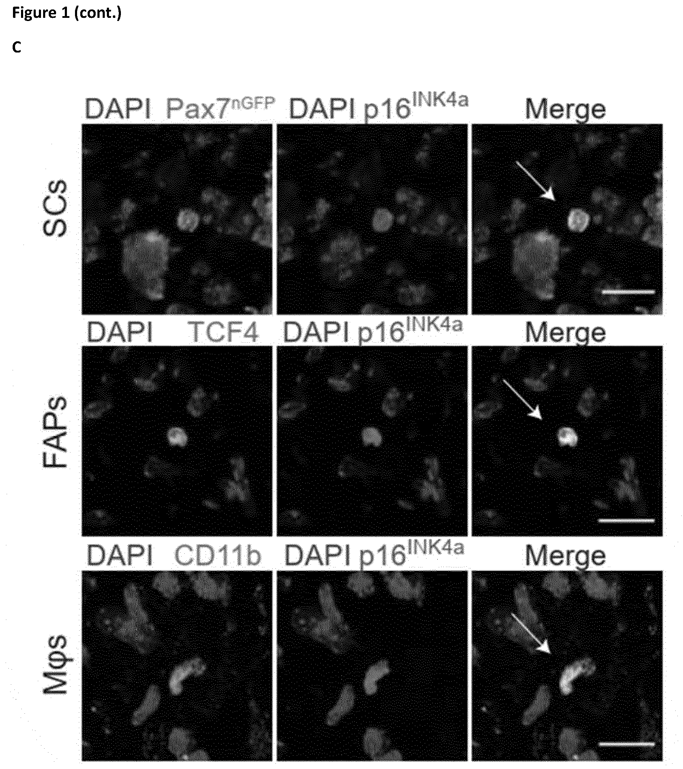

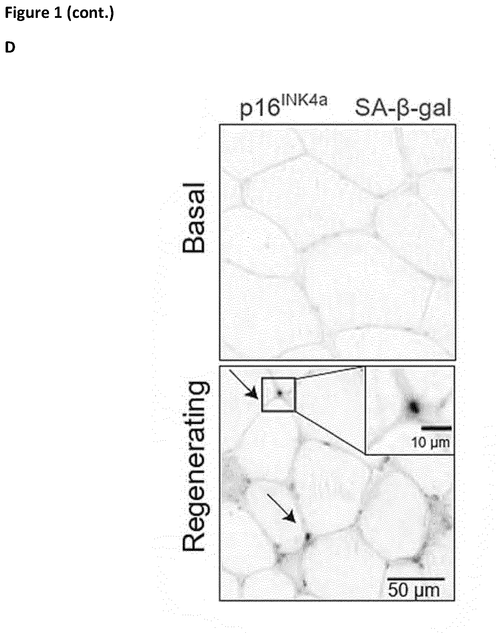

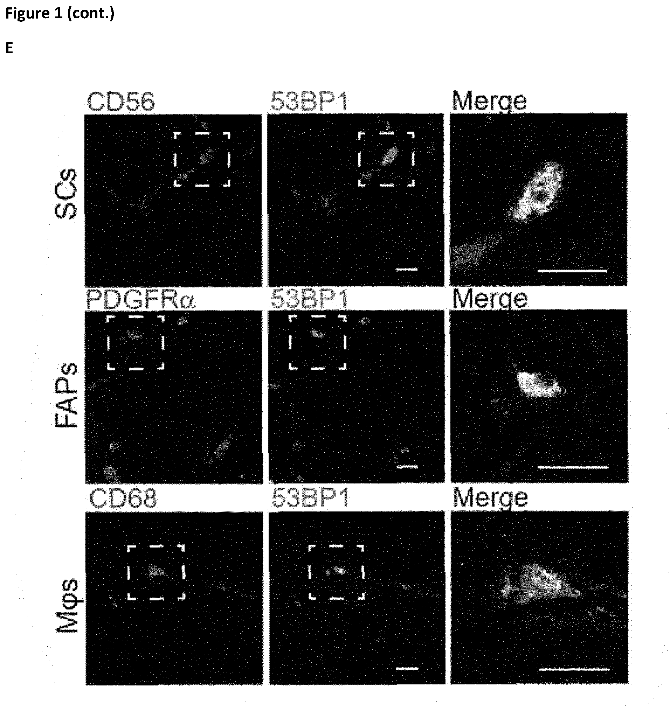

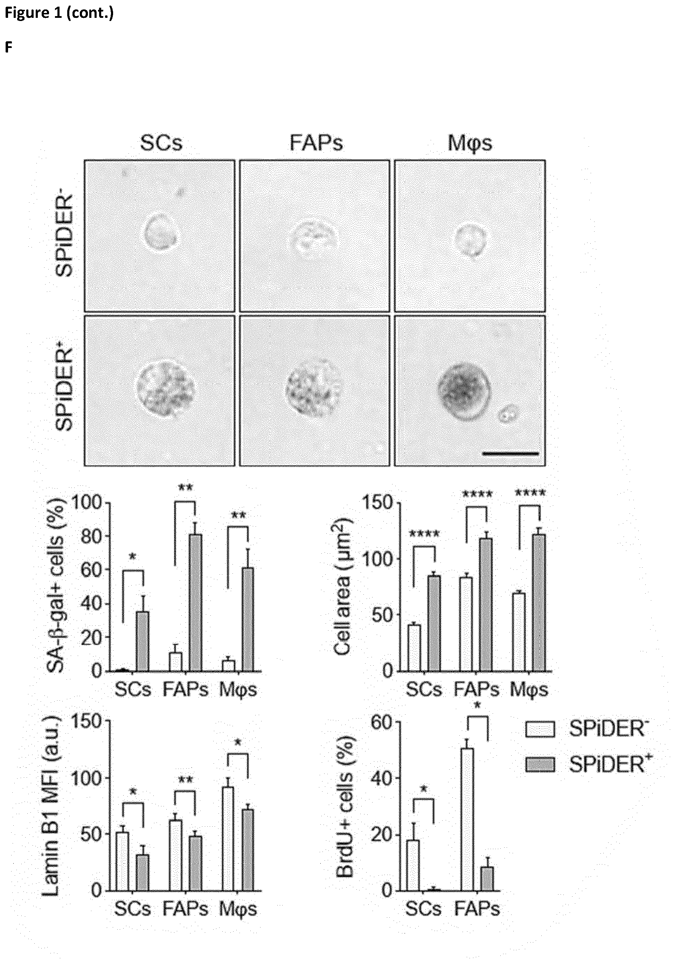

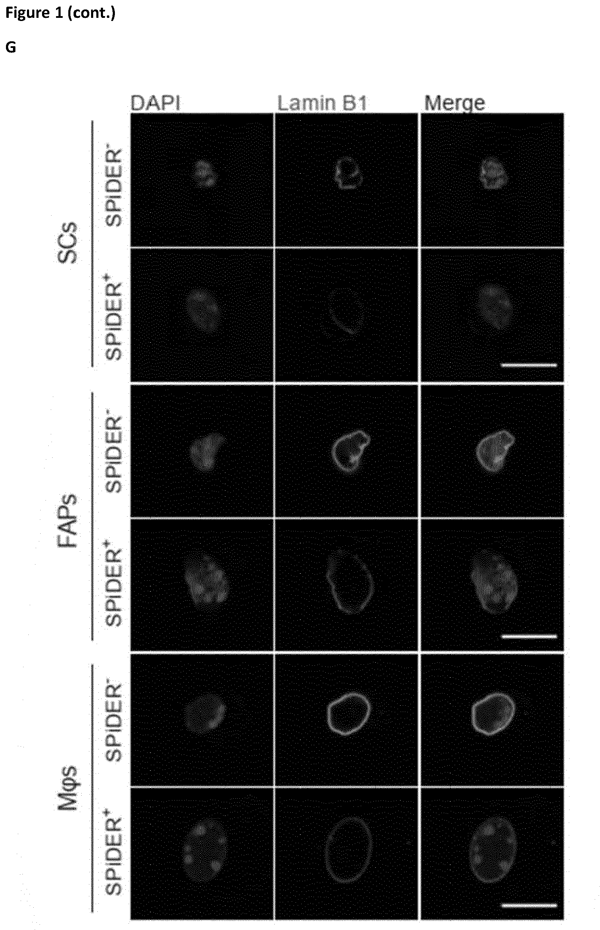

**Figure 1****. Presence of senescent cells in regenerating muscle of young and geriatric mice.** A) Representative images and quantification of *in vivo* Renilla luminescence activity in muscles of p16- 3MR mice at the indicated day post-injury (DPI) with CTX (p/s: photons per second; n=3-18 muscles from 3-12 mice). **B)** Representative images and quantification of SA-β-gal⁺ cells in regenerating TA muscles from young (3-6 months) and geriatric (>28 months) mice at the indicated DPI (n=3-7 mice). **C)** Representative images of p16^{INK4a+} cells in regenerating muscles from young Pax7^{nGFP} and WT mice at 4 DPI. Each cell type was labelled with indicated antibodies, and nuclei with 4,6-diaminido-2-phenylindole (DAPI). Arrows indicate p16^{INK4a+} cells. **D)** SA-β-gal and p16^{INK4a+} immunohistochemistry staining of uninjured and regenerating human muscle. The arrow shows a double-positive cell. **E)** Representative images of 53BP1⁺ cells in regenerating human muscle. Each cell type was labelled with indicated antibodies, and nuclei with DAPI. **F)** Representative images of SA-β-gal staining of freshly sorted SPiDER⁺ and SPiDER⁻ SCs, FAPs, and Mϕs from regenerating muscles at 3 DPI. Quantification of SA-β-gal populations, cell area (n=55-106 cells), Lamin B1 expression (n=15-35 cells; arbitrary units: a.u.) in SPiDER⁺ and SPiDER⁻ sorted populations at 3 DPI. BrdU incorporation was quantified in cells obtained at 7 DPI and cultured for 3 days. **G)** Representative images of Lamin B1 expression in SPiDER⁺ or SPiDER⁻ cells from regenerating muscles at 3 DPI. Scale bars: 50 µm in B and D; 10 µm in C, E and F. Results are displayed as mean ± s.e.m.; P values from two-tailed unpaired t-test; significance is reported as: ^{∗}p<0.05,^{∗∗}p<0.01, ^{∗∗∗}p<0.001 and ^{∗∗∗∗}p<0.0001).

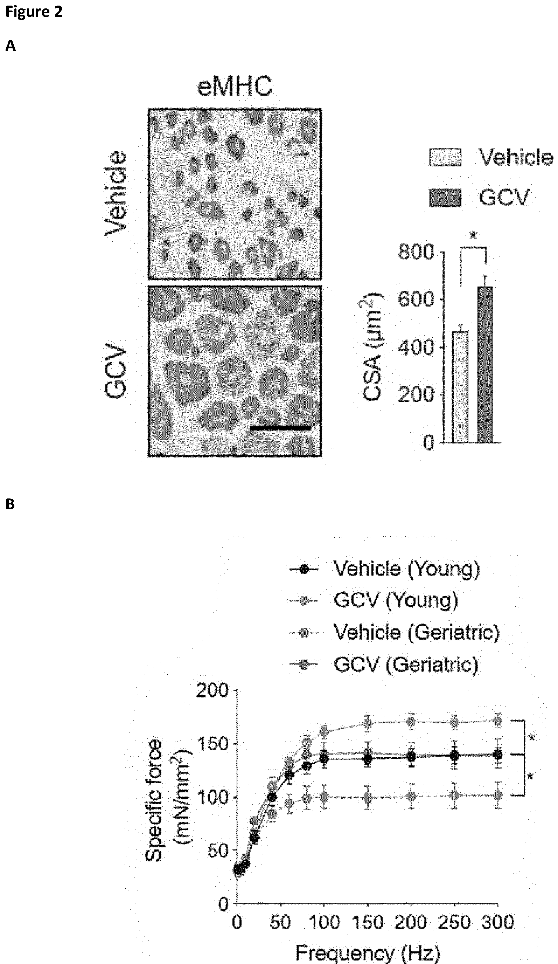

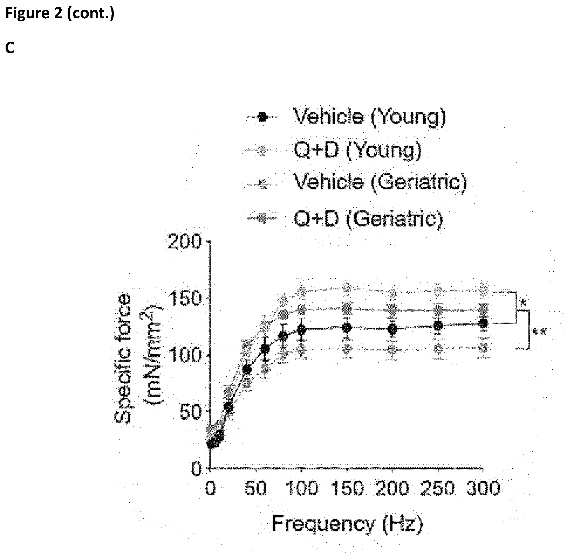

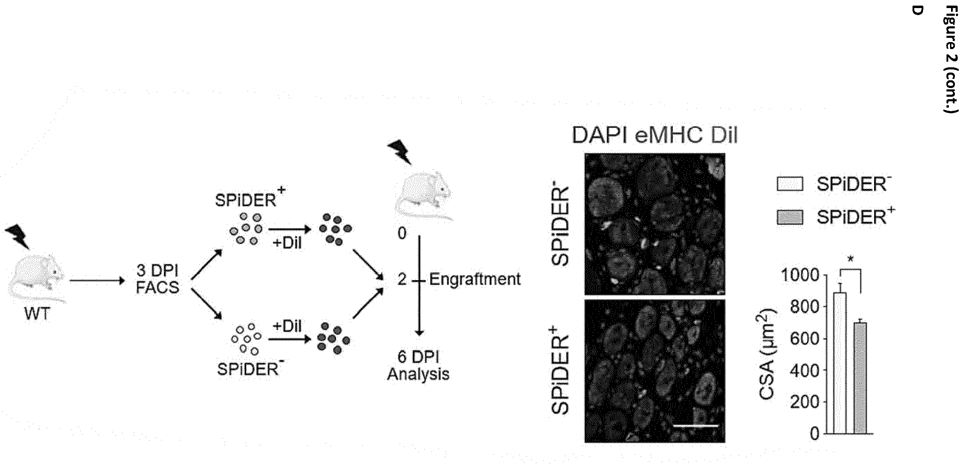

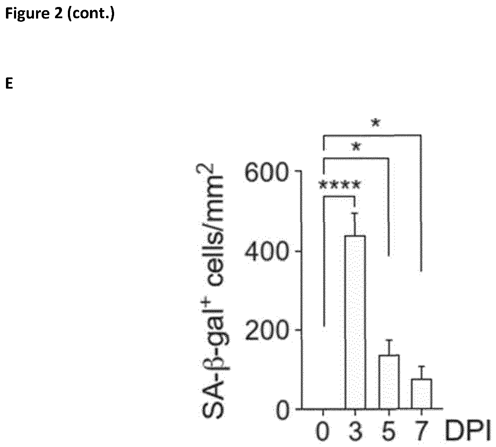

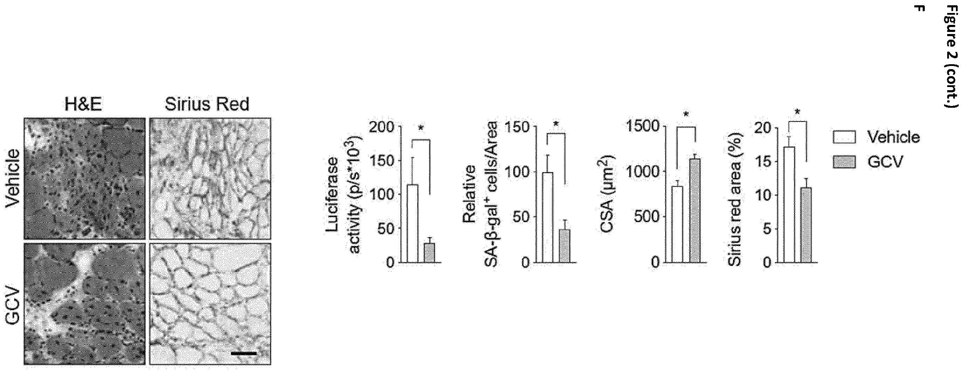

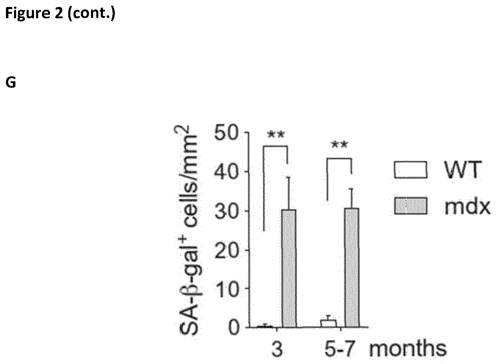

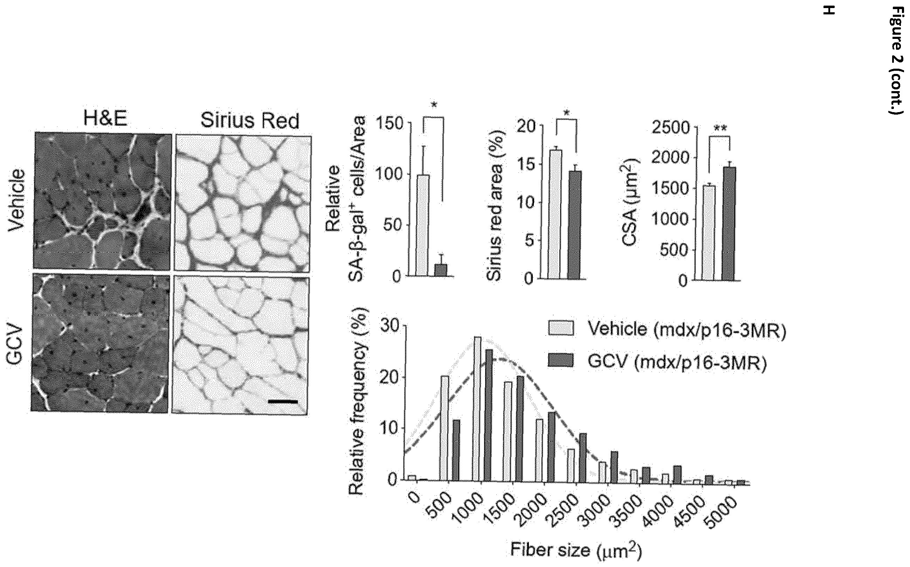

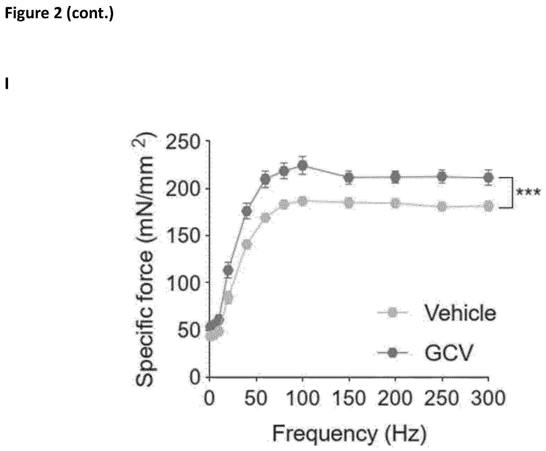

**Figure 2****. Cellular senescence represses muscle regeneration throughout life. A)** Representative images and quantification of cross-sectional area (CSA) of eMHC⁺ fibers in regenerating TA muscles from vehicle- and GCV-treated geriatric p16-3MR mice at 7 DPI (n=5-6 TA from 3 mice). **B)** Force-frequency curves of extensor digitorum longus (EDL) muscles of vehicle- and GCV-treated young and geriatric p16-3MR mice at 10 DPI (n=5-9 EDL from 4-6 mice). **C)** As in B, from vehicle- and Q⁺D⁻ treated mice at 10 DPI (n=6-7 EDL from 3-6 mice). **D)** An equal amount of SPiDER⁺ or SPiDER⁻ cells isolated from young regenerating muscles at 3 DPI were stained with Dil and transplanted into pre-injured TA muscles of young recipient mice for 4 days (n=4 mice). Strategy scheme, representative images, and quantification of CSA of eMHC⁺ fibers are shown. **E)** Quantification of SA-β-gal⁺ cells in TA muscles from young WT mice at indicated days post micropunctures (n=4-6 TA from 3-5 mice). **F)** Young p16⁻ 3MR mice were subjected to micropunctures in TA muscles and treated for 7 days with GCV, starting from the day of injury, sacrificed at 7 DPI and TA muscles were analyzed. Quantification of SA⁻β-gal⁺ cells; *in vivo* Renilla luminescence activity in TA; CSA of central nucleated fibers; Sirius Red staining (n=4-8 muscles from 4 mice). **G)** Quantification of SA⁻β-gal⁺ cells in uninjured TA muscles from WT and mdx mice at indicated age (n=3-7 mice per group). **H)** Young mdx/p16-3MR mice received GCV twice a week for 2 months and were collected at 5 months of age. Representative images of hematoxylin and eosin (H&E) and Sirius Red staining in TA muscles of vehicle- and GCV-treated mdx/p16-3MR mice. Quantifications of SA⁻β-gal⁺ cells; CSA and frequency distribution of regenerating fibers and Sirius Red staining (n=4-6 mice). **I)** Force measurements in EDL muscles of vehicle- and GCV-treated mdx/p16-3MR mice after 2 months of treatment are represented (n=5-9 EDL from 3-6 mice). Scale bars: 50 µm. Values as mean ± s.e.m.; P values were calculated by two-way ANOVA (B, C and I), two-tailed paired t-test (D and E), and Mann-Whitney test for the rest of the graphs; significance is reported as ^{∗}p<0.05, ^{∗∗}p<0.01, ^{∗∗∗}p<0.001.

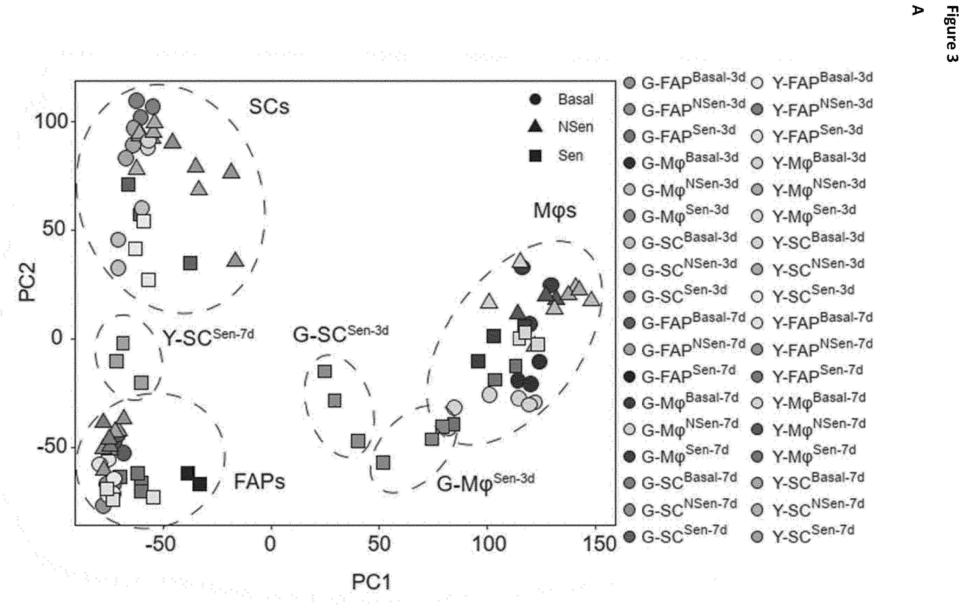

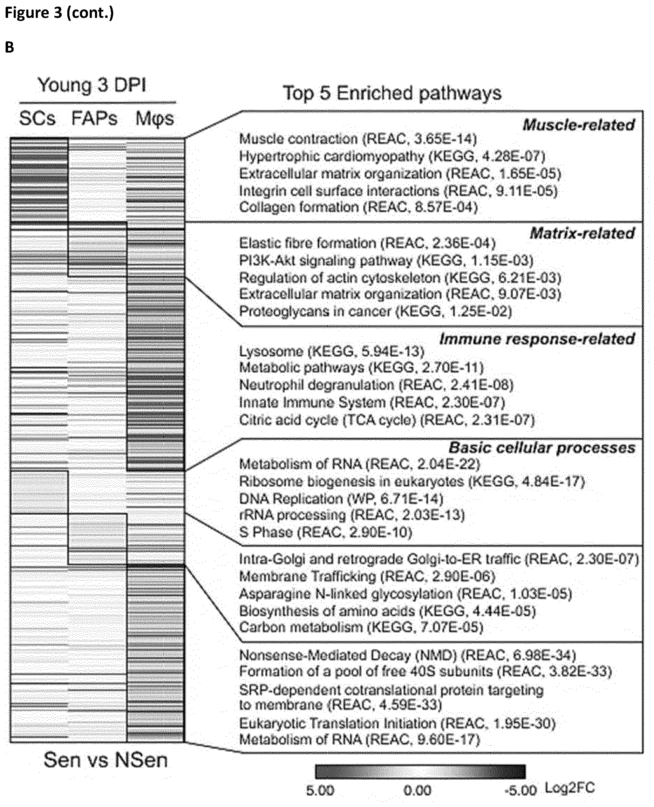

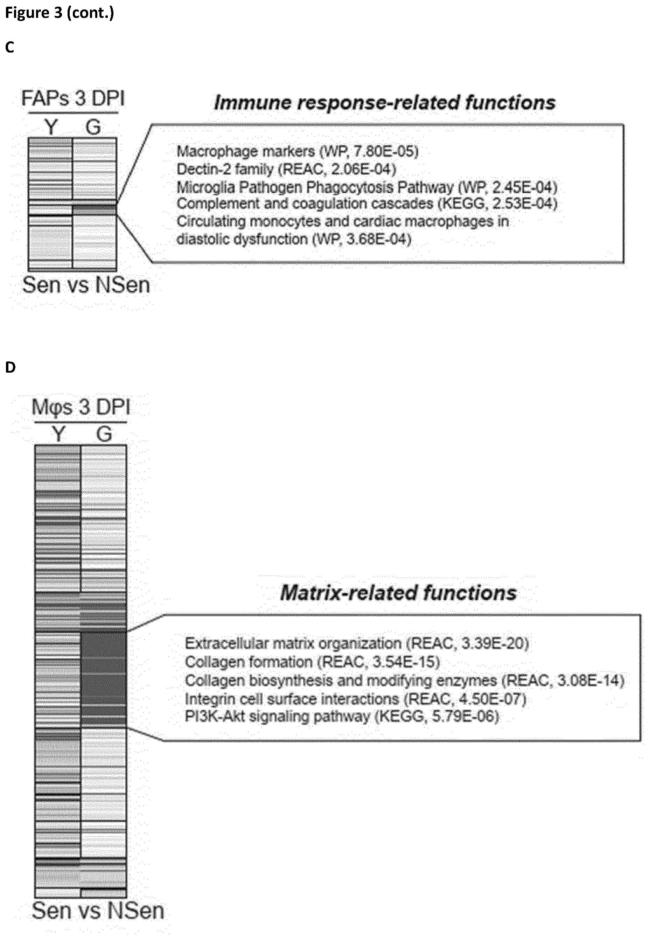

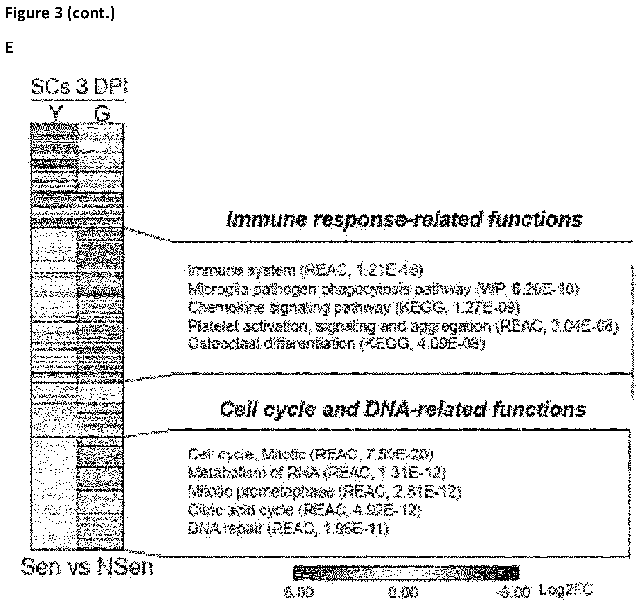

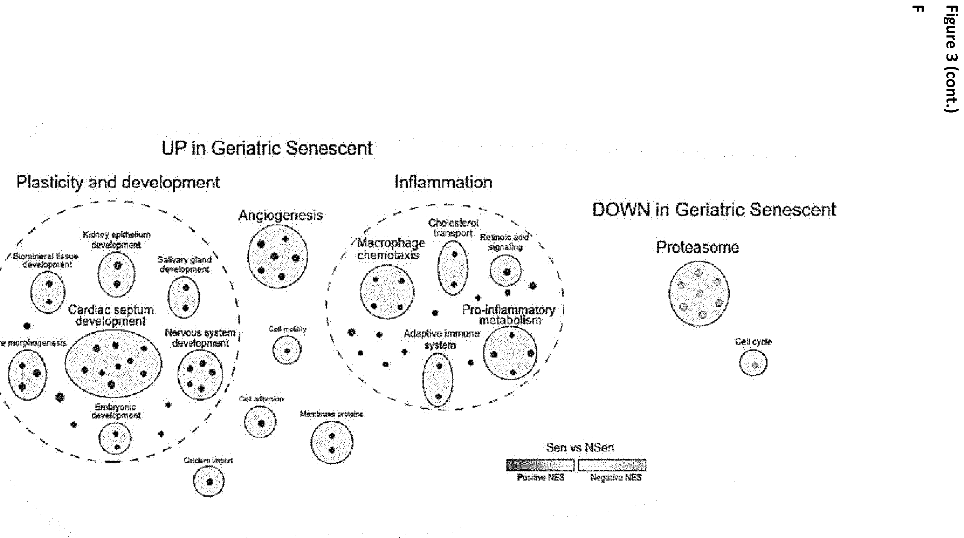

**Figure 3****. Senescent cells maintain expression of cell-type-specific genes and gain expression of lineage-inappropriate genes with aging. A)** Principal component analysis (PCA) of the full transcriptome of senescent (Sen), non-senescent (NSen), and basal SCs, FAPs, and Mϕs isolated from regenerating muscles of young and geriatric mice at 3 and 7 DPI. **B)** Heatmap of genes that were differentially expressed (DE) uniquely by one population of interest and the corresponding canonical pathways enrichment (CP) analysis. The figure shows base 2 logarithm fold change (log2FC) for Sen versus (vs) their NSen counterparts isolated from regenerating muscles of young mice at 3 DPI. **C-E)** As in B, Heatmap of unique and common DE genes in young (Y) or geriatric **(G)** Sen populations and CP enrichment of exclusively DE genes in G populations. **F)** Clusters of gene sets (GSEA) differentially enriched at 3 DPI in geriatric Sen populations, but not in young Sen populations. Gene sets were considered common with FDR<0.25 for all 3 geriatric Sen populations with the exclusion of gene sets common for at least 2 young Sen populations.

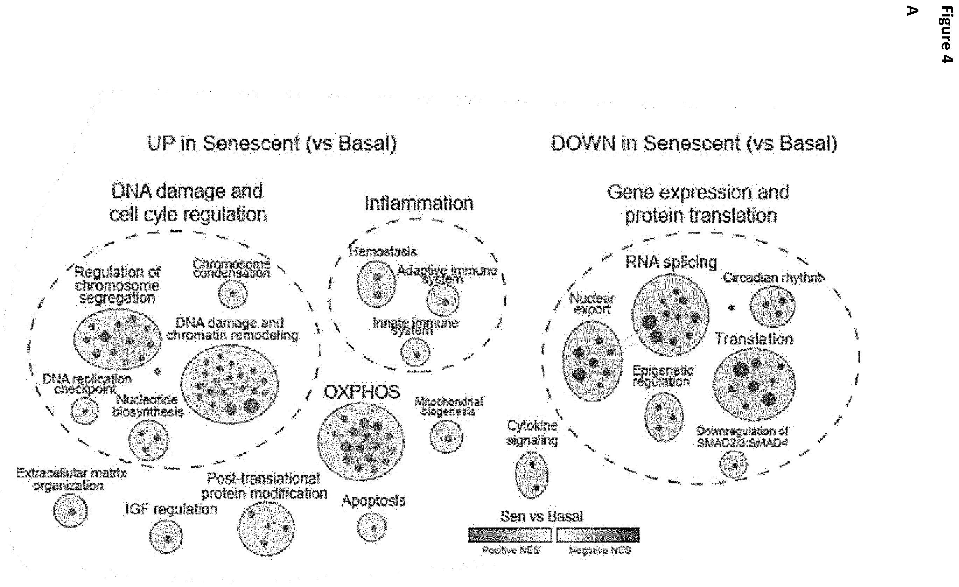

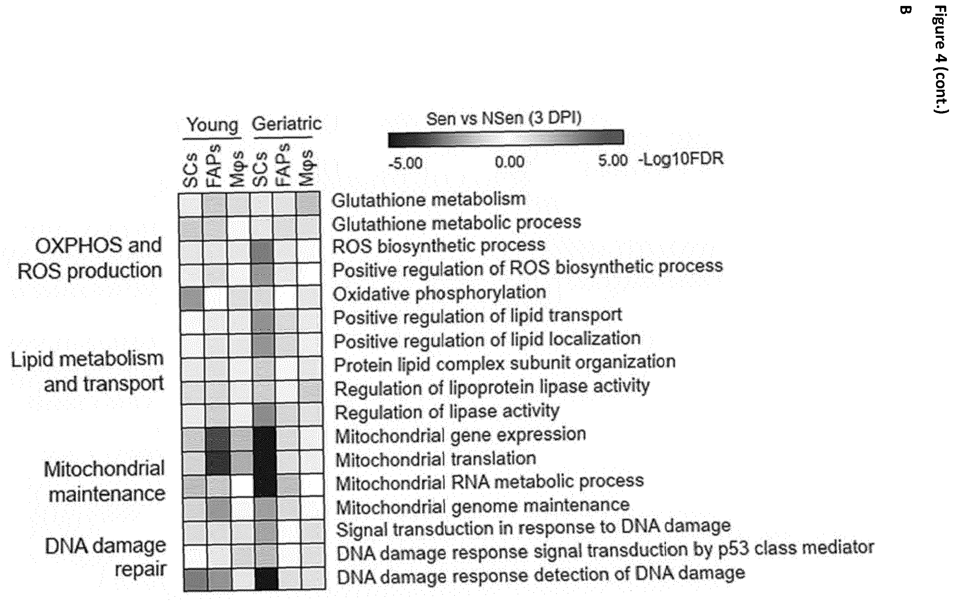

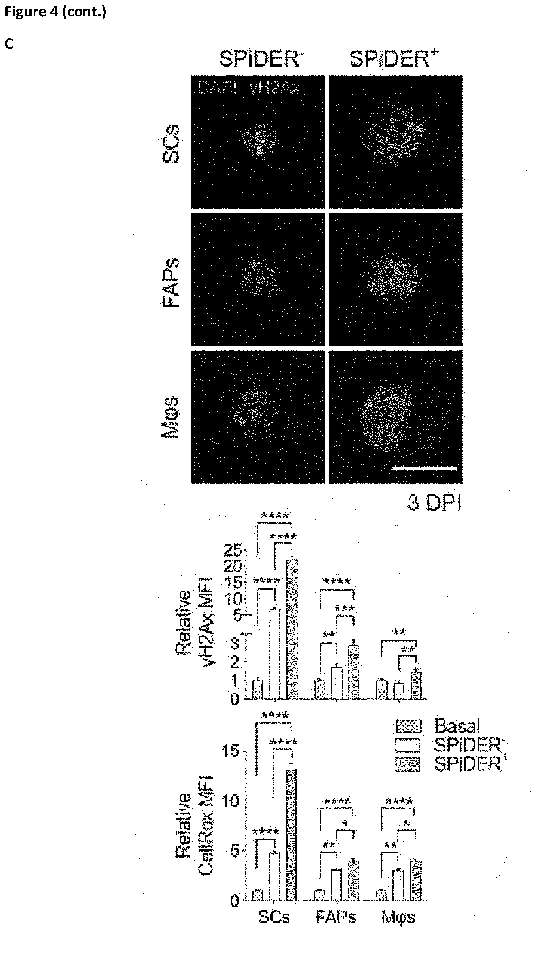

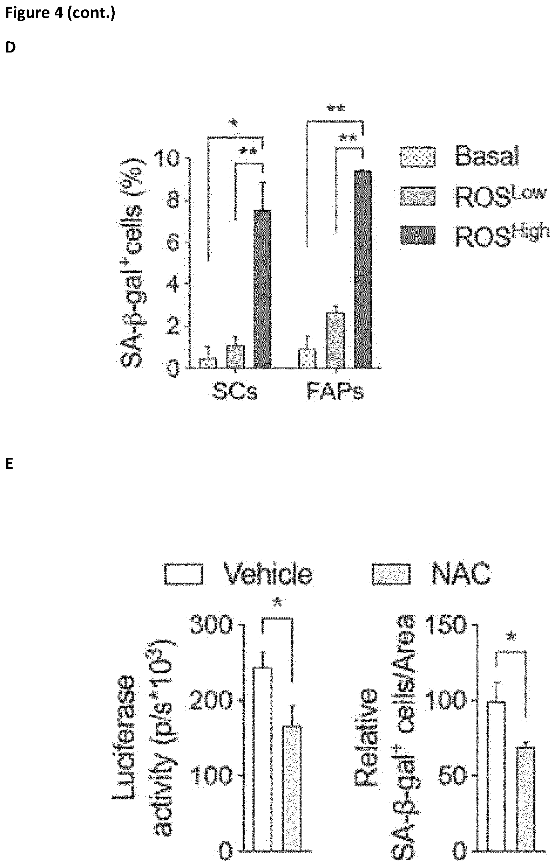

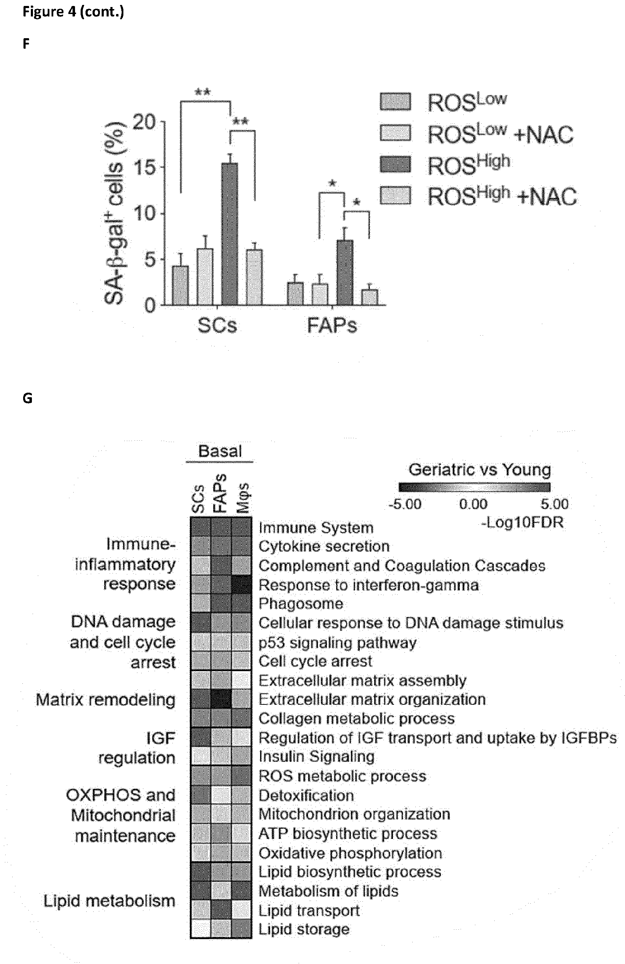

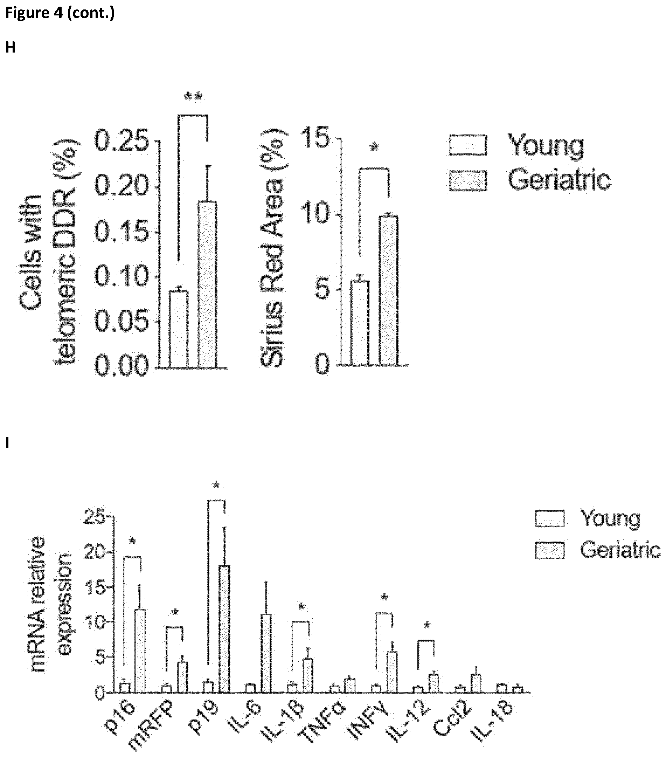

**Figure 4****. Tissue injury and aging separately prime niche cells for senescence by inducing intolerable levels of oxidative stress and DNA damage. A)** Clusters of gene sets (GSEA) differentially enriched from Sen vs Basal SCs, FAPs, and Mϕs from young and geriatric mice at 3 DPI. Gene sets were considered common with FDR<0.25 in at least 5/6 comparisons. **B)** Heatmap of common up-and downregulated pathways (GSEA, FDR<0.25) related to indicated functions in Sen vs NSen SCs, FAPs, and Mϕs from young and geriatric mice at 3 DPI. The figure shows -log2FDR for Sen vs their NSen counterparts. **C)** Representative images of yH2Ax and quantification of yH2Ax (n= 20-91 cells) and CellRox levels (n= 20-104 cells) in freshly sorted SCs, FAPs, and Mϕs populations from Basal and regenerating muscles at 3 DPI. b ROS^{High} and ROS^{Low} SCs and FAPs were isolated from regenerating muscle at 1 DPI and cultured *in vitro* for 3 days. Quantification of SA-β-gal⁺ cells in each population compared to basal cells (n=3 replicates). **E)** Young p16-3MR mice were injured with CTX and treated with NAC in drinking water during regeneration. (Left) Renilla luciferase activity in TA muscles at 4 DPI, (Right) quantification of SA-β-gal⁺ cells (n=5-6 TA from 4 mice). **F)** As in B, cells were sorted from regenerating muscles at 1 DPI, cultured for 3 days with or without NAC *in vitro* (n=3 replicates). **G)** Heatmap of gene sets enriched in DE genes from Geriatric vs Young SCs, FAPs, and Mϕs (FDR≤0,05) isolated from non-injured muscle tissue. **H)** Quantification of cells with telomeric DDR (DDR: DNA damage response) and Sirius Red in TA muscles from young and geriatric mice (n=3-5 mice). **I)** mRNA quantification by RT-qPCR of indicated genes in TA muscles from young and geriatric mice (n=5- 8 mice). Scale bar 10 µm. Results are displayed as mean ± s.e.m.; P values were calculated using Mann- Whitney test (E and H right) and two-tailed unpaired t-test (C, D, F, H left and I); significance is reported as ^{∗}p<0.05, ^{∗∗}p<0.01, ^{∗∗∗}p<0.001 and p<0.0001.

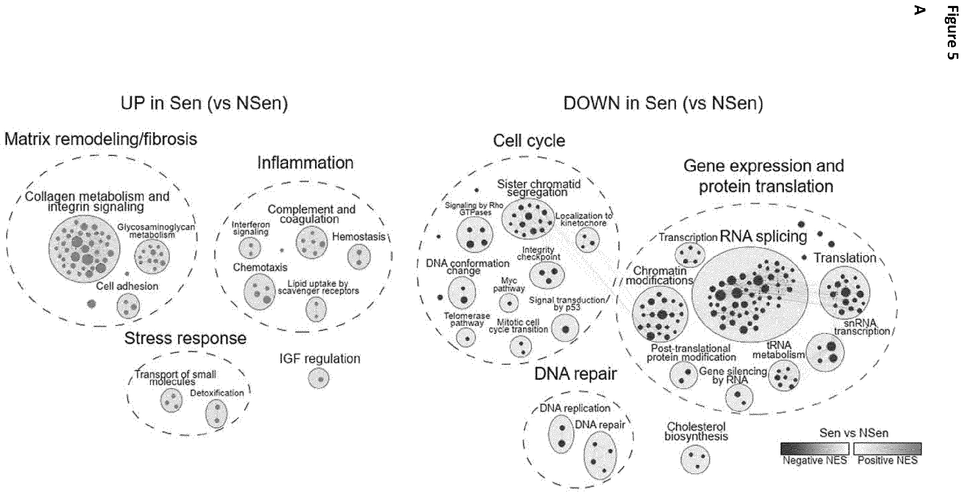

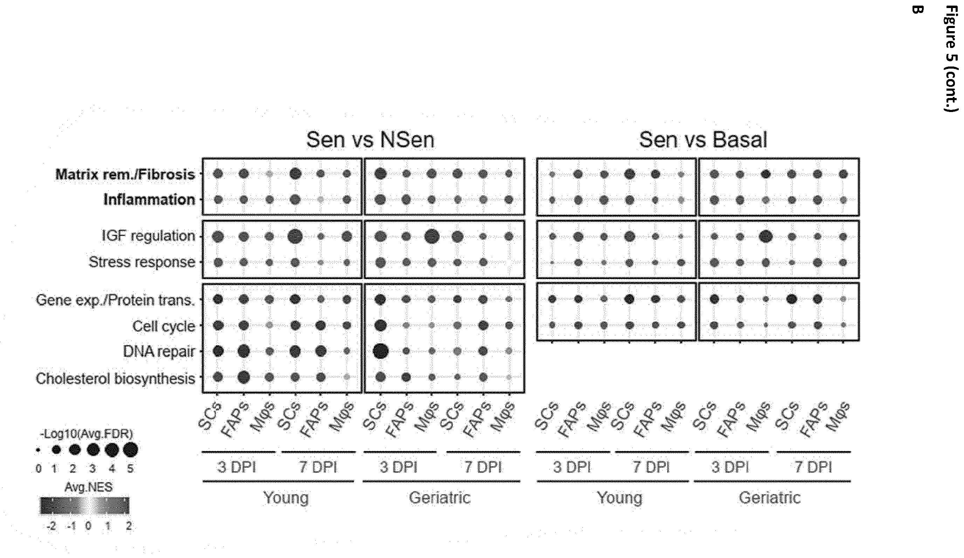

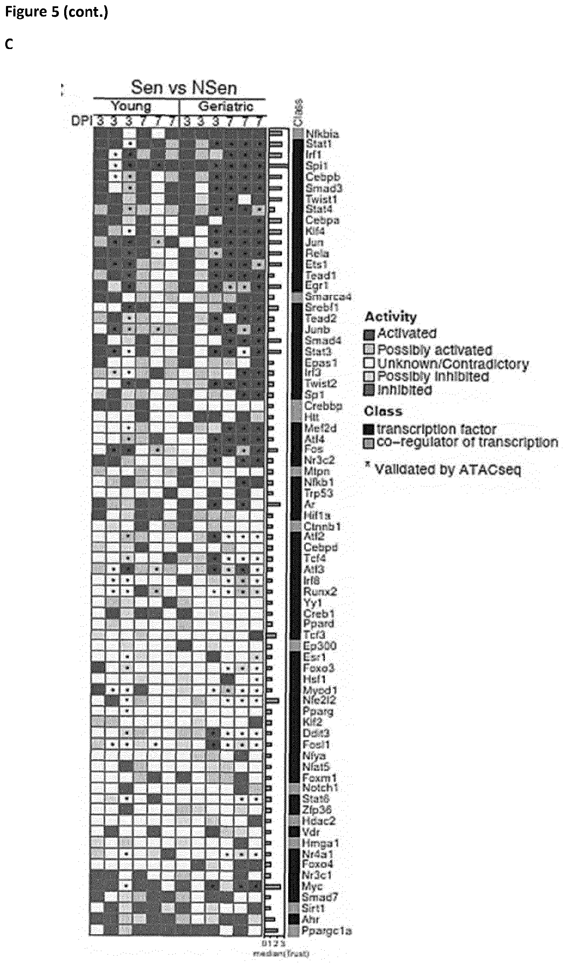

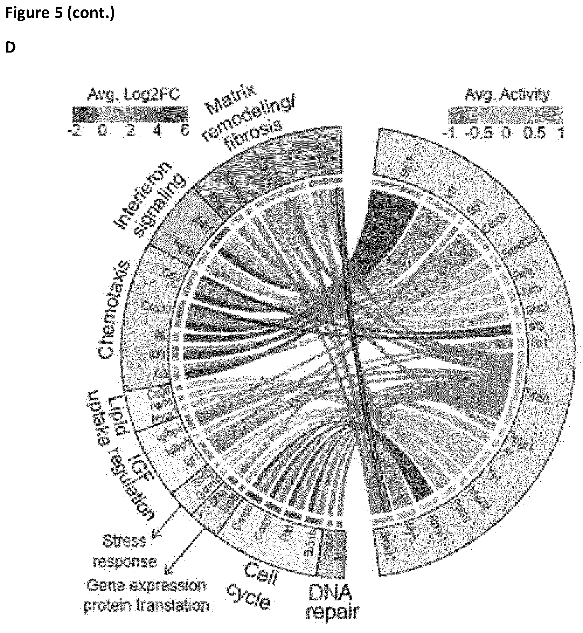

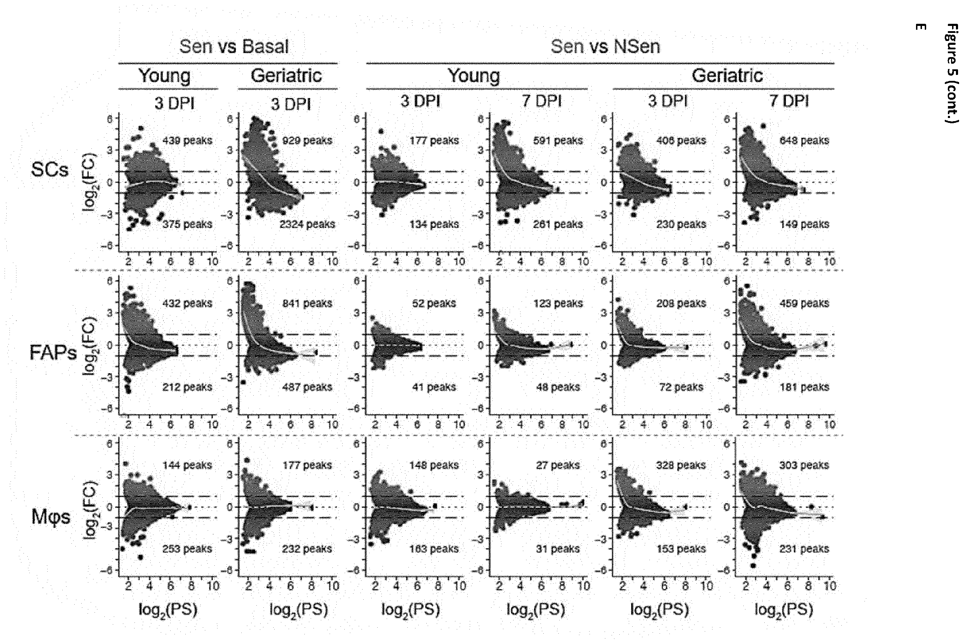

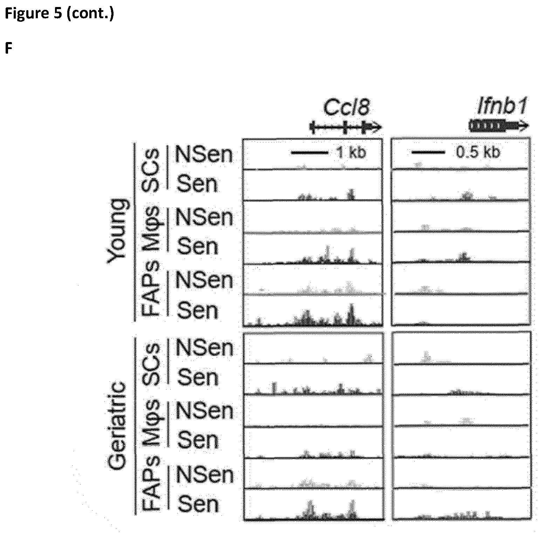

**Figure 5****. Two major common hallmarks define senescent cells across cell types, stages of regeneration, and lifespan. A)** Clusters of gene sets (GSEA) differentially enriched from Sen vs NSen SCs, FAPs, and Mϕs from young and geriatric mice at 3 and 7 DPI. Gene sets were considered common with exploratory FDR< 0,25 in at least 8/12 comparisons. **B)** Dot plot representing common clusters of gene sets (GSEA) from Sen vs NSen and Sen vs Basal SCs, FAPs, and Mϕs from young and geriatric mice at 3 and 7 DPI. Gene sets were considered common with exploratory FDR<0,25 in at least 8/12 comparisons for Sen vs NSen and Sen vs Basal. **C)** Heatmap representing transcription factors and co-regulators of transcription enriched in at least 11/12 comparisons for Sen vs NSen with average Trust score > 1 (see methods). Color codes reflect the activity predicted based on analysis of differential expression (DESeq2), upstream regulators analysis (QIAGEN's IPA), and motif enrichment analysis in RNAseq and ATACseq data. **D)** Chord diagram showing TFs regulating the DE genes in Sen vs NSen and their respective categories. Chord width is proportional to the -log of the average minimum FDR for CP and GO:BP enrichment (gprofiler2) within a given functional category. Green-to-orange scale indicates average predicted TF activity. **E)** Differential ATAC-seq peaks for Sen vs NSen SCs, FAPs, and Mϕs from geriatric mice at 3 and 7 DPI. Left: log2FC is plotted against log2-transformed average peak intensity (peak score); the greem line shows the position of each average peak score on the log2FC axis; the number of peaks with log2FC >1 or <-1 is indicated. **F)** Normalized ATAC-seq signal profiles in the indicated gene regions from Sen vs NSen SCs, FAPs, and Mϕs from young and geriatric mice at 7 DPI.

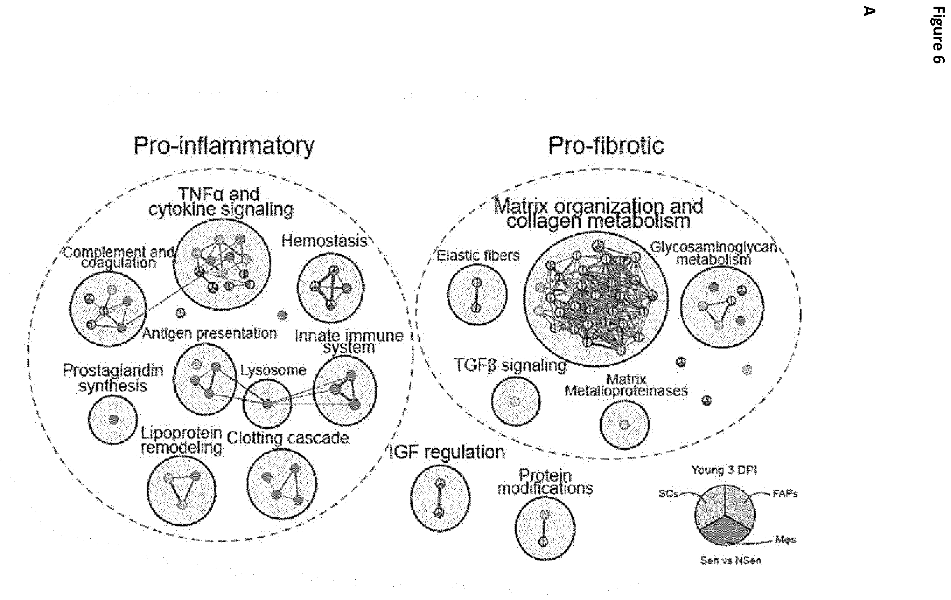

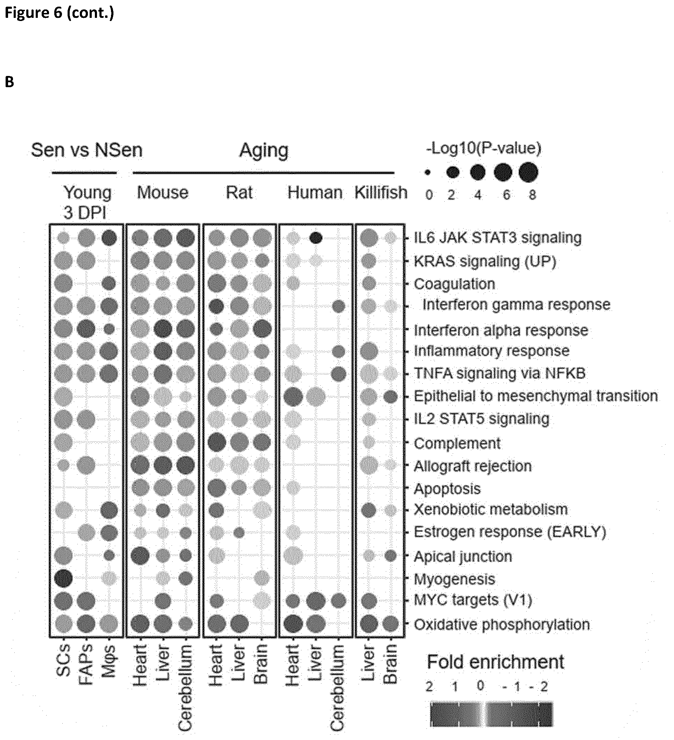

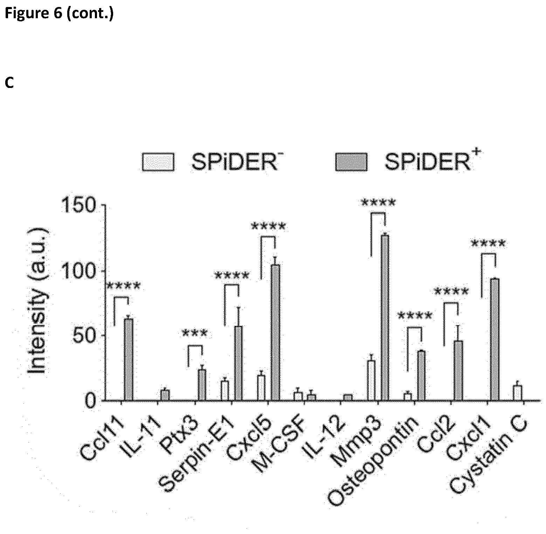

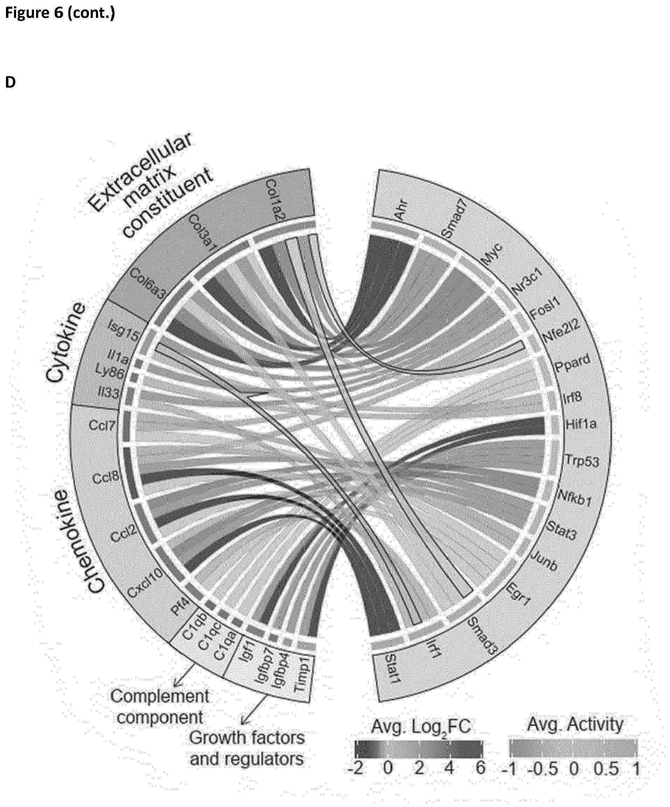

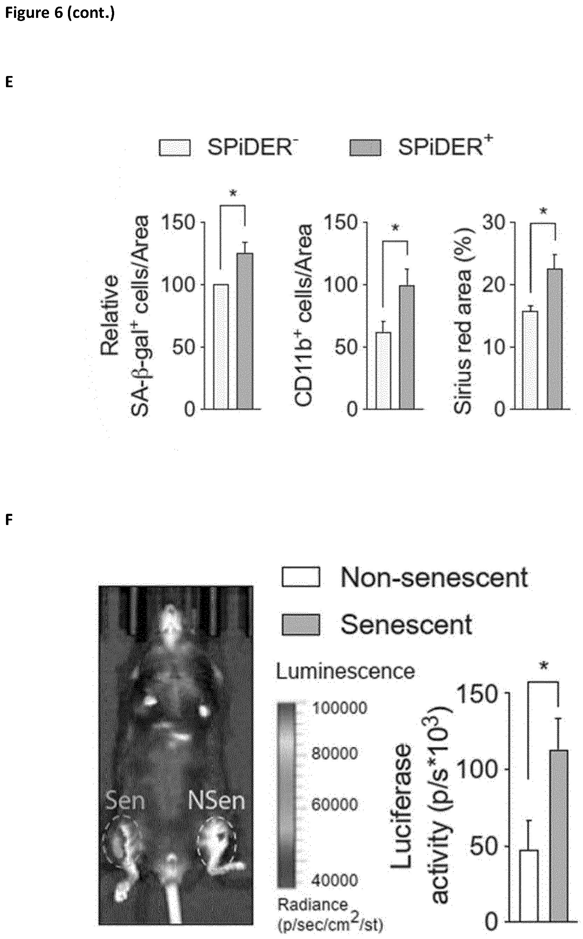

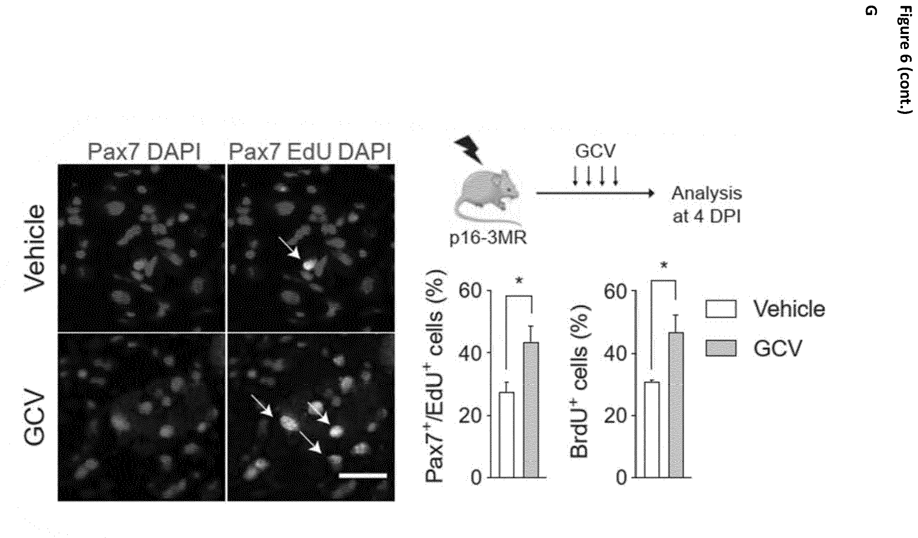

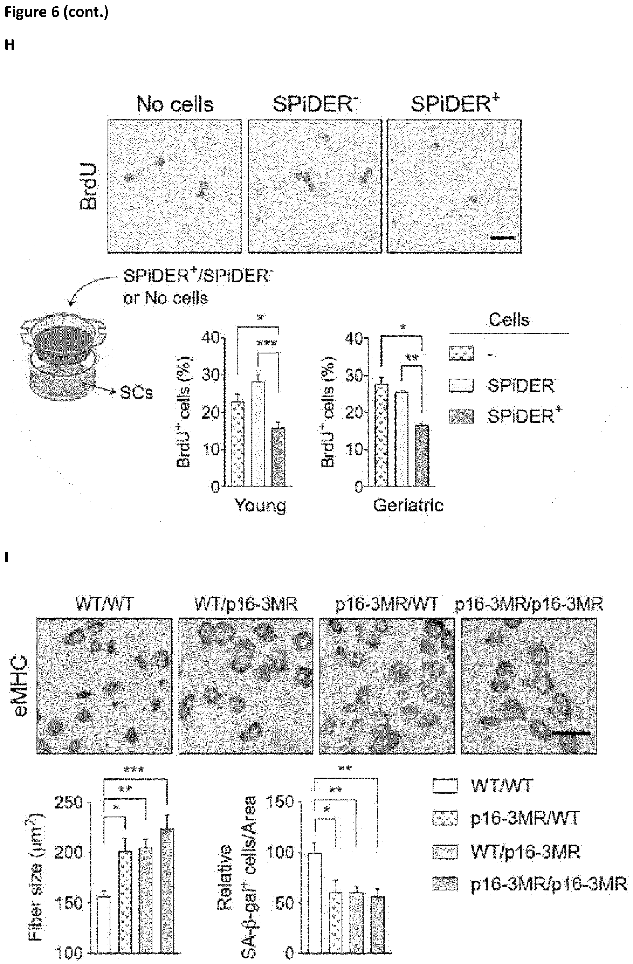

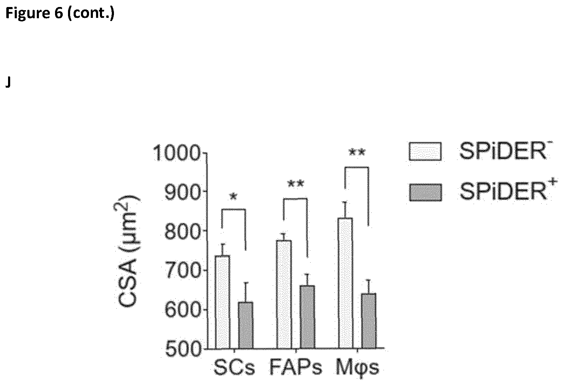

**Figure 6****. Senescent cells create an aged-like microenvironment in young regenerative niches through the secretion of pro-inflammatory and pro-fibrotic factors. A)** Clusters of gene sets enriched in SASP-related genes from Sen SCs, FAPs, and Mϕs from young mice at 3 DPI (g:Profiler web server, FDR<0.05) (see methods). Differentially upregulated genes (FDR<0.05) were considered as "SASP genes" when overexpressed in Sen populations vs their NSen counterparts. **B)** Dot plot comparing functional enrichments of MSigDB 5.1 hallmarks obtained by using the minimum hypergeometric test for differential RNA expression in different tissues from aged mice, rats, humans, killifish and muscle senescent populations from young mice at 3 DPI. **C)** Cytokine array of freshly sorted SPiDER⁺ or SPiDER⁻ cells from regenerating muscle at 3 DPI cultured for 24 hours in serum-deprived media, then the conditioned media was collected, and the levels of the indicated protein were assessed. **D)** Chord diagram showing TFs regulating the SASP genes and their respective categories in Sen vs NSen. Chord width is proportional to the -log of the average p-value for GO:MF cluster enrichment (see methods). **E)** SPiDER⁺ or SPiDER⁻ cells isolated from young regenerating muscle at 3 DPI were stained with Dil and transplanted into pre-injured TA muscle of the recipient young mice for 4 days (n=4 mice). Quantification of SA-β-gal⁺ cells, CD11b⁺ cells, and Sirius Red staining. **F)** Senescent and non- senescent C2C12 cells were stained with Dil and transplanted into pre-injured TA muscle of the recipient young p16-3MR mice for 5 days. Representative images and quantification of *in vivo* Renilla luminescence activity in muscles of p16-3MR mice (n=5 mice). **G)** p16-3MR mice were injured with CTX and daily treated with vehicle or GCV from the day of injury to 4 DPI (n=3-6 TA from 3 mice). (Left) representative images of EdU or Pax7 staining and quantification of EdU incorporation in SCs of TA. (Right) quantification of BrdU incorporation in SCs *in vitro.* At 3 DPI, in mice treated as before, SCs were FACS⁻ sorted and cultured for 3 days (n=3 biological replicates, from 3 mice). **H)** SPiDER⁺ or SPiDER⁻ SCs were isolated at 3 DPI from regenerating muscles of young/geriatric mice, then cultured for 3 days in transwells with total SPiDER⁺ or SPiDER⁻ cells or medium. After 3 days of culture, SCs proliferation was assessed by BrdU incorporation. Representative images and quantification of BrdU⁺ cells (n=3 replicates). **I)** EDL from either WT or p16-3MR donor mice were transplanted into WT or p16-3MR recipient mice or vice versa. Recipient mice were treated with GCV and regeneration was assessed 7 days later. Representative images, and quantification of fiber size and SA-β-gal⁺ cells (n=6-8 mice). **J)** SPiDER⁺ or SPiDER⁻ cells isolated from young regenerating muscles at 3 DPI were stained with Dil and transplanted into pre-injured TA muscle of the recipient young mice for 4 days. Quantification of CSA of eMHC⁺ fibers and SA-β-gal⁺ cells (n=4-5 mice). Scale bars: 20 µm in G and I; 10 µm in H. Results are displayed as mean ± s.e.m.; P values from Mann-Whitney test (E, G left and I), two-tailed unpaired t-test (C and G right), two-tailed paired t- test (F, J) and two-way ANOVA (H); significance reported as ^{∗}p<0.05, ^{∗∗}p<0.01, ^{∗∗∗}p<0.001,^{∗∗∗∗}p<0.0001.

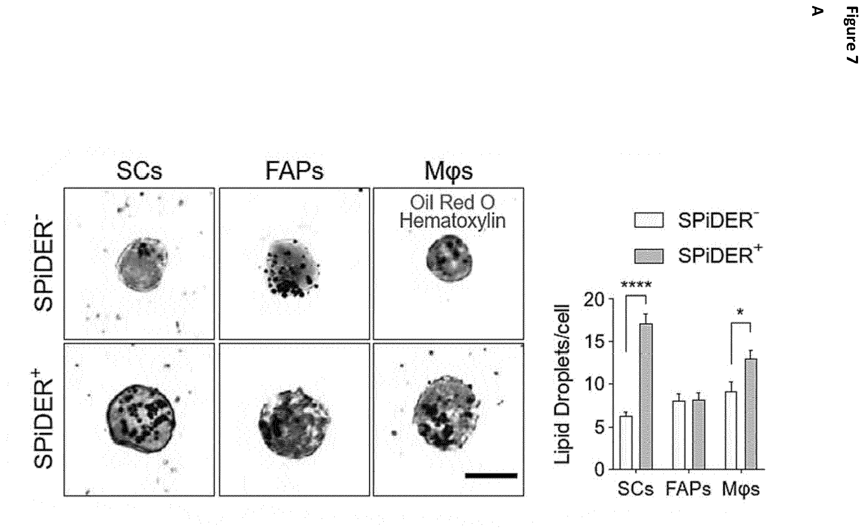

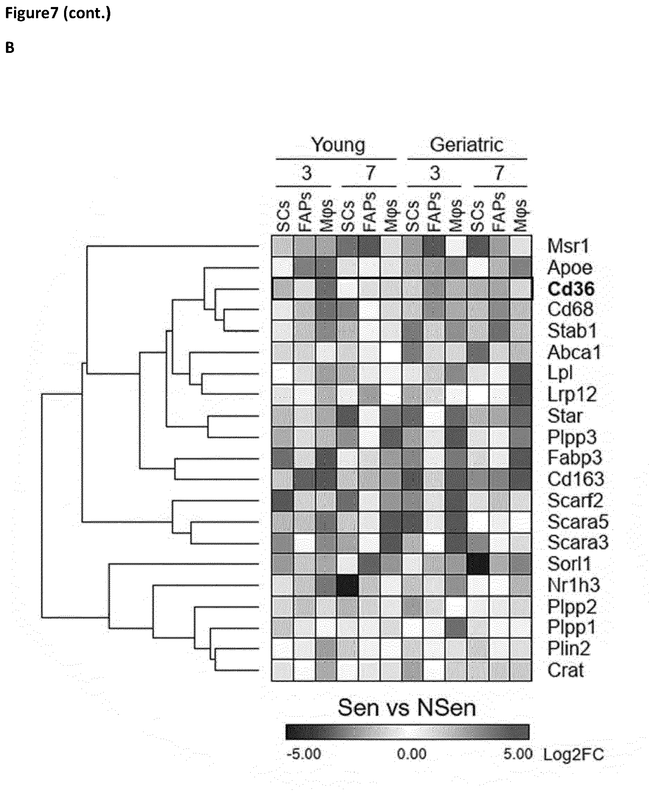

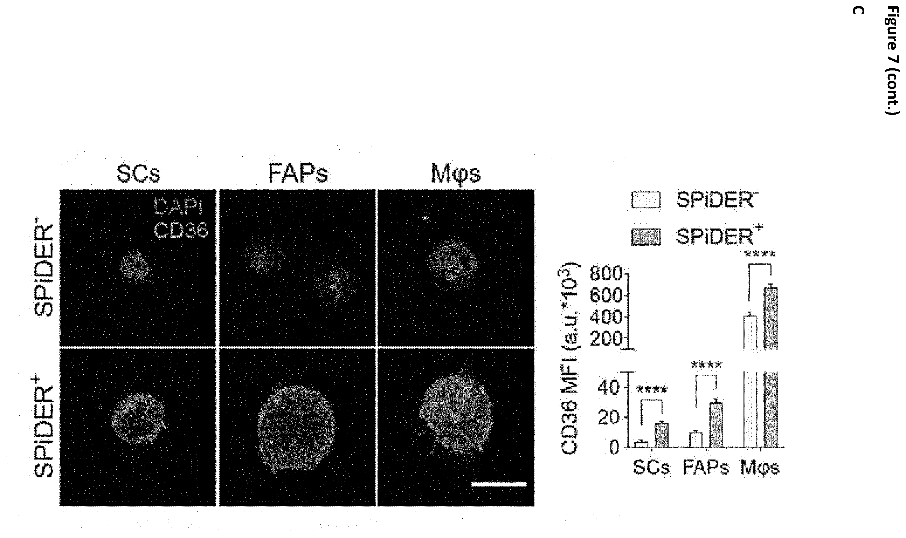

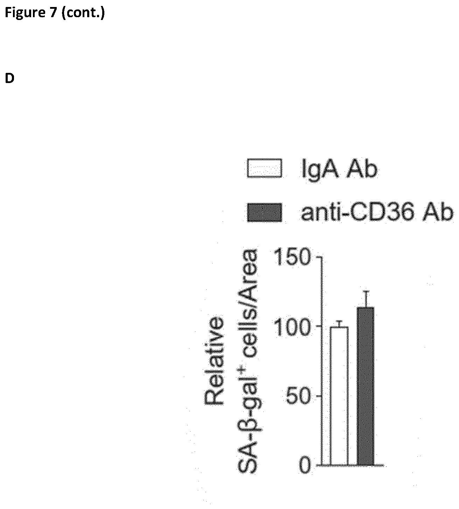

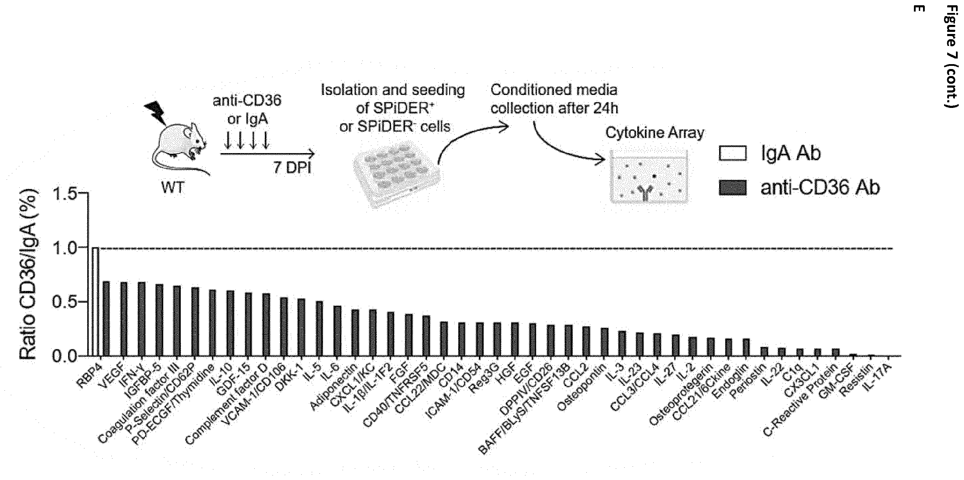

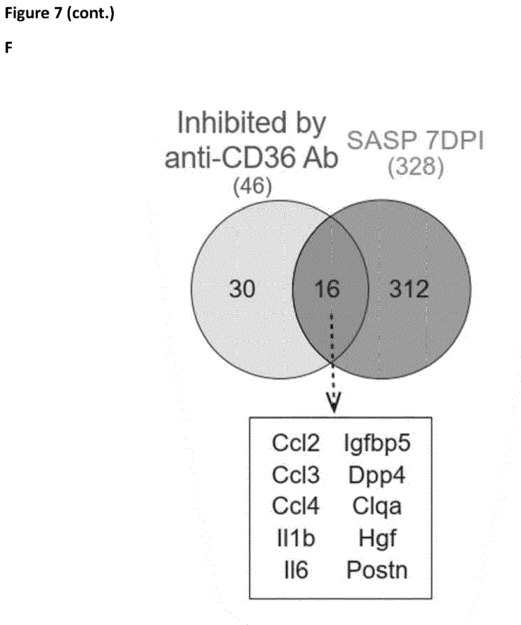

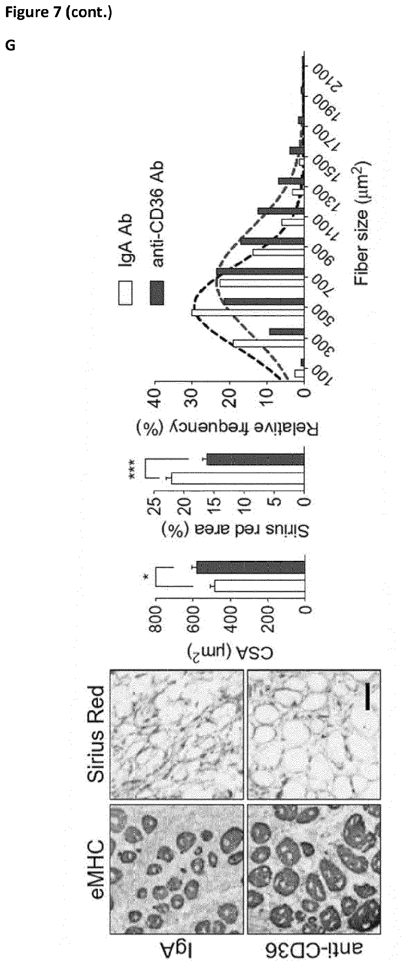

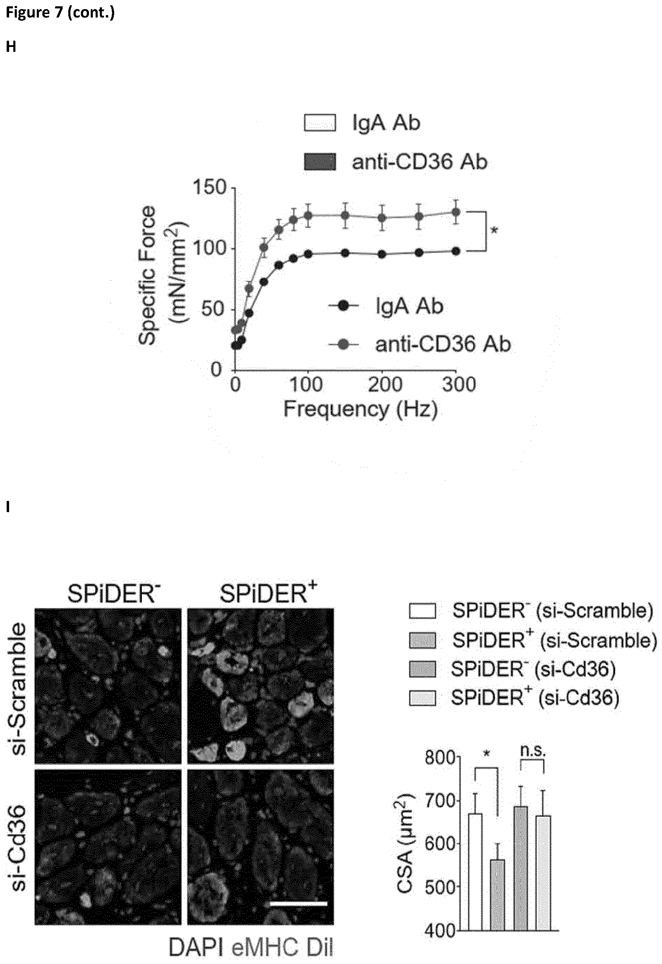

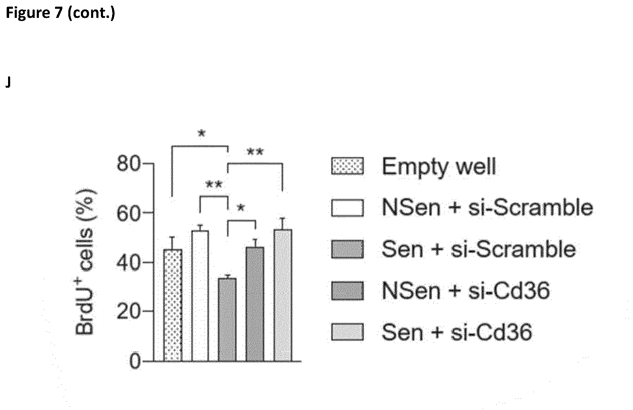

**Figure 7****. CD36 neutralization improves regeneration in young and old mice through a senomorphic action. A)** SPiDER⁺ and SPiDER⁻ populations from regenerating muscle at 3 DPI were stained with Oil Red O and hematoxylin. Representative images and quantification of lipid droplets are shown (n= 45-94 cells). **B)** Heatmap showing lipid-transport related genes that were DE in at least 3/12 comparisons between Sen and their NSen counterparts. The color indicates log2FC of gene expression. **C)** Representative images and quantification of CD36 levels in freshly sorted SPiDER⁺ and SPiDER⁻ populations from regenerating muscles at 3 DPI (n= 46-62 cells). **D)** Quantification of SA-β-gal⁺ cells in damaged area of TA muscles from aged mice (20-month-old) injured with CTX and treated with control anti-IgA or anti-CD36 antibody from 3 to 7 DPI once per day (n=8 TA from 4 mice). **E)** Freshly sorted SPiDER⁺ cells from IgA⁻ or anti-CD36 antibody-treated old mice at 7 DPI were cultured for 24 hours in serum-deprived media, conditioned media was collected, and protein levels assessed by cytokine array. (Up) Scheme of the experimental design. (Down) Quantification showing the proteins whose levels were reduced by 30% in the presence of anti-CD36 antibody. The dotted line indicates levels in IgA-treatment. **F)** Venn diagram showing the overlap between SASP-related upregulated genes in senescent populations isolated from geriatric mice at 7 DPI and genes codifying for proteins reduced by anti-CD36 antibody treatment in E. **G)** As in E, quantification of CSA and frequency distribution analysis of eMHC⁺ fiber size and Sirius Red staining of regenerating TA muscles (n=7-8 TA from 4 mice). **H)** EDL muscles of old mice were injured with CTX and mice were treated with anti-IgA or anti-CD36 antibody from 3 to 10 DPI (n=5 EDL from 3 mice). Force-frequency curves of are shown. **I)** SPiDER⁺ or SPiDER⁻ cells isolated from young regenerating muscles at 3 DPI were transfected with si-Scramble or si-Cd36, stained with Dil, and transplanted into pre-injured TA muscle of the recipient young mice. Samples were collected 4 days after transplantation. Representative images and quantification of CSA of eMHC⁺ fibers, and SA-β-gal⁺ cells in damaged areas (n=6-8 mice). **J)** SCs were isolated at 3 DPI from regenerating muscles of young mice, then cultured during 3 days in transwells with C2C12 cells or without adding cells. After 3 days of culture, SCs proliferation was assessed by BrdU incorporation. Representative images and quantification of BrdU⁺ cells (n=3 replicates). Scale bar 10 µm (A and C) and 50 µm (I). Results are mean ± s.e.m.; P values were calculated by two-tailed unpaired t-test (A and C), Mann-Whitney test (G) and two-way ANOVA (H), one-way ANOVA (J) and two-tailed paired t-test (I); significance is reported as ^{∗}p<0.05, ^{∗∗∗∗}p<0.0001.

### Detailed description of the invention

The present invention is illustrated by means of the Examples set below, without the intention of limiting its scope of protection.

### Example 1. Material and methods

### Example 1.1. Experimental Model and Subject Details Animals models

C57B1/6 (WT), p16-3MR (kindly donated by J. Campisi) (Demaria et al., 2014), dystrophic DBA/2-mdx, DBA/2-mdxp16-3MR (dystrophic DBA/2-mdx mice crossed with p16-3MR mice), and Pax7^{nGFP} were bred and aged at the animal facility of the Barcelona Biomedical Research Park (PRBB), housed in standard cages under 12-hour light-dark cycles and fed ad libitum with a standard chow diet. The Catalan Government approved the work protocols, following applicable legislation. Both male and female mice were used in each experiment unless stated otherwise. Live colonies were maintained and genotyped as per Jackson Laboratories' guidelines and protocols. Mice were housed together, health was monitored daily for sickness symptoms (not age-related weight loss, etc), and euthanized immediately at the clinical endpoint when recommended by veterinary and biological services staff members. No statistical methods were used to predetermine the sample size.

### Example 1.2. Human biopsies

Muscle biopsies from human adult subjects were obtained via the Tissue Banks for Research from Vall d'Hebron and Sant Joan de Deu Hospitals and especially via the EU/FP7 Myoage Consortium. Muscle biopsies were taken from the vastus lateralis muscle under local anesthesia (2% lidocaine). A portion of the muscle tissue was directly frozen in melting isopentane and stored at -80 °C until analyzed.

### Example 1.3. In vivo treatments

ABT-293 (Active Biochem, #A-1001; 100 mg/kg), quercetin (USP, #1592409; 100 mg/kg or 50 mg/kg when co-administered with dasatinib) and dasatinib (LC Laboratories, #D-3307; 10 mg/kg or 5 mg/kg when co-administered with quercetin) were administered orally (gavage). Control mice were administered with an equal volume of vehicle (10 % ethanol, 30 % polyethilenglicol, and 60 % phosal). Ganciclovir (GCV, Sigma-Aldrich, #G2536-100MG; 25 mg/kg) and 17-DMAG (LC Laboratories, #D-3340 ;10 mg/kg) were injected intraperitoneally (i.p.). Anti-CD36 antibody (Cayman Chemical, #10009893; 10 µg or 20 µg in young and 20 µg in old mice) diluted in PBS was administered via i.p., control mice received an equal dose of IgA control antibody (Southern Biotech/Bionova, #0106-14). Treatments with GCV, senolytics, and CD36 were administered daily for 4-7 consecutive days as indicated in the figure legends. N-acetylcysteine (NAC, Sigma-Aldrich, #A9165; 0.01 g/ml) was added into drinking water (exchanged every three days) one week before muscle injury and was prolonged until sacrifice. SIS3 (Sigma-Aldrich, #S0447-5MG; 10 mg/kg) in PBS and bortezomib (Teva, #TH000345/LF2.3; 0.5 mg/kg) in 10% DMSO diluted in PBS were administered i.p. (from 1 to 5 DPI). For long term treatments, 3 months old 2-mdx/DBA and 2-mdx/DBA^{p16-3MR} mice were administered with quercetin plus dasatinib (Q⁺D) or GCV respectively twice a week for 2 months.

### Example 1.4. Muscle regeneration

Mice were anesthetized with ketamine-xylazine (80 and 10 mg/kg respectively; i.p.) or isoflurane. Regeneration of skeletal muscle was induced by intramuscular injection of cardiotoxin (CTX, Latoxan, #L8102; 10 µM) as described in (Suelves et al., 2007). At the indicated times post-injury, mice were euthanized, and muscles were dissected, frozen in liquid-nitrogen-cooled isopentane, and stored at -80°C until analysis.

### Example 1.5. Heterografting

Heterografting experiments were performed, according to the protocol described in (Grounds et al., 2005). Briefly, the extensor digitorum longus (EDL) muscle was removed from the anatomical bed of either p16- 3MR or WT and was transplanted onto the surface of the tibialis anterior (TA) muscle of the p16-3MR or WT recipient mouse or vice versa. Muscle grafts were collected at day 7 after transplantation.

### Example 1.6. Muscle force measurement

Ex vivo force measurements of EDL muscles was assessed as previously described (Del Prete et al., 2008). Briefly, mice were sacrificed, and muscles were immediately excised and placed into a dish containing oxygenated Krebs-Henseleit solution. Muscles were mounted vertically in a temperature-controlled chamber and immersed in a continuously oxygenated Krebs-Ringer bicarbonate buffer solution, with 10 mM glucose. One end of the muscle was linked to a fixed clamp, while the other end was connected to the lever-arm of a force transducer (300B, Aurora Scientific) using a nylon thread. The optimum muscle length (Lo) was determined from micromanipulations of muscle length to produce the maximum isometric twitch force. The maximum isometric-specific tetanic force was determined from the plateau of the curve of the relationship between specific isometric force with a stimulation frequency ranging from 1 to 300 Hz. Force was normalized per muscle area, determined by dividing the muscle mass by the product of length and muscle density of (1.06 mg/mm3), to calculate the specific force (mN/mm2).

### Example 1.7. p16-3MR Renilla luciferase reporter assay

*In vivo,* renilla luciferase activity was measured in TA, quadriceps (QA), and gastrocnemius (GC) muscles from p16-3MR mice. Anesthetized mice were injected intramuscularly with coelenterazine H (PerkinElmer, #760506) and immediately subjected to measure with the IVIS Lumina III (PerkinElmer). *In vitro,* Renilla luciferase activity was measured from cryopreserved diaphragm and TA muscles using the Dual-Luciferase Reporter Assay Kit (Promega Corporation, #E1910). Signal was measured with the luminometer Centro LB 960 (Berthold Technologies GmbH & Co. KG) and values were normalized to total protein extracted measured by Bradford method (Protein Assay, Bio-Rad, #500-0006) and damaged area measured after H/E staining.

### Example 1.8. Cell isolation by flow cytometry

Muscles were mechanically disaggregated and incubated in Dulbecco's Modified Eagle's medium (DMEM) containing liberase (Roche, #177246) and dispase (Gibco, #17105-041) at 37°C with agitation for 1-2 hours. When required, SPiDER-β-gal reagent (Dojindo, #SG02; 1 µM) was added during the second hour. The supernatant was then filtered, and cells were incubated in lysis buffer (BD Pharm Lyse, #555899) for 10 min on ice, resuspended in PBS with 2.5% fetal bovine serum (FBS), and counted. BV711-conjugated anti- CD45 (BD, #563709; 1/200), APC-Cy7-conjugated anti F4/80 (Biolegend, #123118; 1/200), PE-conjugated anti-α7-integrin (Ablab, #AB10STMW215; 1/200), APC-conjugated anti-CD31 (eBioscience, #17-0311-82; 1/200) and PE-Cy7-conjugated anti-Sca-1 (Biolegend, #108114; 1/200) antibodies were used to isolate Mϕs (CD45⁺ and F4/80⁺), SCs (α7-integrin⁺, CD45⁻, F4/80⁻ and CD31⁻) and FAPs (Sca-1⁺, CD45⁻, F4/80⁻, α7-integrin⁻ and CD31⁻). SPiDER-β-gal (SPiDER) was employed to isolate senescent cells (SPiDER⁺) from non-senescent cells (SPiDER⁻) of each cell type. Cells were sorted using a FACS Aria II (BD). Isolated cells were used either for RNA extraction, cell cultures, engraftments, proliferation assays or plated on glass slides (Thermo Scientific, #177402) for immunostaining and SA-β-gal analysis. To isolate ROS^{High} and ROS^{Low} populations, digested muscle was stained with CellRox Green reagent (Invitrogen, #C10444; 5 µM) according to manufacturer's protocol and PE-Cy7-conjugated anti-CD45 (Biolegend, #103114; 1/200), PE-Cy7-conjugated anti-CD31 (Biolegend, #102418; 1/200), PE-conjugated anti-α7-integrin (Ablab, #AB10STMW215; 1/200), and APC-conjugated anti-Sca-1 (Biolegend, #108111; 1/200) antibodies to separate SCs (α7-integrin⁺, CD45⁻ and CD31⁻) and FAPs (Sca-1⁺, CD45⁻, α7-integrin⁻ and CD31⁻). CellRox^{High} and CellRox^{Low} cells were sorted using a FACS Aria II (BD). Isolated cells were used for cell cultures and proliferation assays.

### Example 1.9. Senescent cells transplantation

Cells transplants were performed as in (Sousa-Victor et al., 2014), following an adapted protocol (Sacco et al., 2010). FACS-isolated SPiDER⁺ and SPiDER⁻ cells were collected, resuspended in 20% FBS DMEM medium, labeled with Vybrant Dil Cell Labelling solution (Invitrogen, #V22889) according to manufacturer instructions and injected into TA muscles of recipient WT mice previously injured with the freeze crush method two days before (Le et al., 2016). Each TA muscle was engrafted with 10.000 cells, except when each senescent cell type was transplanted separately (see **Figure 6J**), where 5.000 cells were engrafted. Engrafted muscles were collected and processed for muscle histology 4 days after cell transplantation.

### Example 1.10. RNA interference

Freshly sorted cells or C2C12 cells were transfected with siRNA targeting Cd36 (On-Target plus SmartPool, Dharmacon, #L-062017-00-0005; 5 nM) or unrelated sequence as control (On-Target plus non- targeting siRNA Pool, Dharmacon, #D-001810-10-05; 5 nM) using the DharmaFect protocol (Dharmacon, #T-2003-02).

Target sequences for Cd36 siRNA were:
5'-CCACAUAUCUACCAAAAUU-3' (SEQ ID NO: 1)
5′- GAAAGGAUAACAUAAGCAA-3' (SEQ ID NO: 2)
5'-AUACAGAGUUCGUUAUCUA-3' (SEQ ID NO: 3)
5'-GGAUUGGAGUGGUGAUGUU-3' (SEQ ID NO: 4)

Freshly sorted cells after incubation with siRNAs for three hours cells were washed and engrafted.

### Example 1.11. Cytokine array

Cytokine antibody array (R&D Systems, #ARY028) was used according to the manufacturer's protocol. Briefly, freshly sorted cells were cultured for 24h in serum-free DMEM. Cell culture supernatants were collected, centrifuged and incubated with the membranes precoated with captured antibodies. Then, membranes were incubated with detection antibodies, streptavidin-HRP, and Chemi Reagent Mix. The immunoblot images were captured and visualized using the Chemidoc MP Imaging System (Bio-Rad, Hercules, USA) and the intensity of each spot in the captured images was analyzed using the publicly available ImageJ software.

### Example 1.12. Proliferation assays

To assess proliferation *in vivo,* muscles were injured by local CTX injection, and mice were administered with ethynyl-labelled deoxyuridine (EdU, Invitrogen, #A10044; 25.5 mg/kg; i.p.) two hours before the sacrifice at 3-4 DPI. Muscles were collected and processed for immunofluorescence staining in tissue slides or cell isolation by FACS. Edu-labeled cells were detected using the Click-iT EdU Imaging Kit (Invitrogen, #C10086). EdU-positive cells were quantified as the percentage of the total number of cells analyzed. *In vitro* proliferation was quantified on freshly sorted SCs, which were seeded in 20% FBS Ham's F10 medium supplemented with b-FGF (Peprotech, #100-18B-250UG; 2.5 ng/ml) in collagen-coated plates and FAPs cultured in 20% FBS DMEM supplemented with b-FGF (Peprotech, #100-18B-250UG; 2.5 ng/ml). After 3 days of culture, SCs and FAPs were pulse-labeled with bromodeoxyuridine (BrdU, Sigma-Aldrich, #B9285-1G; 1.5 µg/ml) for 1 h. BrdU-labeled cells were detected by immunostaining using rat anti-BrdU antibody (Abcam, #AB6326; 1:500) and a specific secondary biotinylated donkey anti-rat antibody (Jackson Inmunoresearch, #712-066-150; 1:250). Antibody binding was visualized using Vectastain Elite ABC reagent (Vector Laboratories, #PK-6100) and 3,3'-Diaminobenzidine (DAB). BrdU-positive cells were quantified as the percentage of the total number of cells analyzed.

### Example 1.13. Transwell assay

SCs were freshly isolated from regenerating muscle tissue at 3 DPI and plated on 24-well plates (Falcon, #353047) in 20 % FBS DMEM supplemented with b-FGF. Subsequently, medium or freshly sorted SPiDER⁺ and SPiDER⁻ cell populations or etoposide-induced senescent C2C12 cells were seeded on 0.4 µm pore size cell culture insert (Falcon, #353495) using the same medium. After 3 days of culture, proliferation assay was performed on SCs with BrdU labeling as described above.

### Example 1.14. In vitro treatments

ROS^{High} and ROS^{Low} SCs and FAPs were freshly isolated from regenerating muscle at 24 hours post-injury, seeded, and cultured in presence of NAC (10 mM) or vehicle for 3 days. After the treatment, cells were fixed and further processed for staining. C2C12 cells maintained in 10% FBS DMEM, were treated with etoposide (Sigma-Aldrich, #E1383, 1 µM) for 5 days to induce senescence and collected for RNA extraction and RT-qPCR. Cells were stained with β-galactosidase staining kit (as described below) to confirm their senescent state.

### Example 1.15. Cell staining

SA-β-galactosidase (SA-β-gal) activity was detected in freshly sorted cells and cell cultures using the senescence β-galactosidase staining kit (Cell signalling, #9860), according to the manufacturer's instructions. Lipid droplets were stained with Oil Red O (Sigma-Aldrich, #O0625) according to manufacturer instructions. ROS levels were measured by immunofluorescence using CellRox Green reagent (Invitrogen, #C 10444; 5 µM) according to instructions. ImageJ software was used to perform image analysis.

### Example 1.16. Muscle histology, immunofluorescence and immunoFISH

Muscles were embedded in OCT solution (TissueTek, #4583), frozen in isopentane cooled with liquid nitrogen, and stored at -80 °C until analysis. 10 µm muscle cryosections were collected and stained for SA- β-gal (AppliChem, #A1007,0001), hematoxylin/eosin (H/E, Sigma-Aldrich, #HHS80 and #45235), eMHC (DSHB, #F1.652), Sirius Red (Sigma-Aldrich, #365548) or used for immunofluorescence (see **Table 1**).

**Table 1**

| **Antibody** | **Brand** | **Reference** | **Dilution** |
|---|---|---|---|
| nGFP | Invitrogen | #A6455 | 1/400 |
| eMHC | DSHB | #F1.652 | Ready to use |
| p16^{INKA4} | Invitrogen | #MA5-17142 | 1/100 |
| TCF4 | Cell Signaling | #2569S | 1/80 |
| CD11b | eBioscience | #14-0112-85 | 1/100 |
| Lamin B 1 | Abcam | #ab16048-100 | 1/100 |
| CD36 | Invitrogen | #MA5-14112 | 1/100 |
| γH2AX | Cell Signaling | #2577S | 1/50 |
| Pax7 | Abcam | #ab34360 | 1/20 |
| Ki67 | Abcam | #ab15580 | 1/100 |

CSA on H/E and eMHC stained sections, percentage of muscle area positive for Sirius Red staining, and number of SA-βGal⁺ were quantified using Image J software. Double immunofluorescence was performed by the sequential addition of each primary and secondary antibodies using positive and negative controls. The sections were air-dried, fixed, washed on PBS, and incubated with primary antibodies according to the standard protocol after blocking with a high-protein-containing solution in PBS for 1 h at room temperature. Subsequently, the slides were washed with PBS and incubated with the appropriate secondary antibodies and labeling dyes. Telomere immunoFISH was performed after yH2AX immunofluorescence staining with Telomeric PNA probe (Panagene, #F1002-5) as described (Fumagalli et al., 2012).

### Example 1.17. Digital image acquisition

Digital images were acquired using: an upright DMR6000B microscope (Leica) with a DFC550 camera for immunohistochemical color pictures; a Thunder imager 3D live-cell microscope (Leica Microsystems) with AFC (hardware autofocus control) and a Leica DFC9000 GTC sCMOS camera, using HC PL FLUOTAR
× 10/0.32 PH1 ∞/0.17/ON257C and HC PL FLUOTAR ×20/0.4 CORR PH1 ∞/0-2/ON25/C objectives; a Zeiss Cell Observer HS with a ×20 and x40 air objective and Zeiss AxioCam MrX camera; and a Leica SP5 confocal laser-scanning microscope with HCX PL Fluotar ×40/0.75 and ×63/0.75 objectives; the different fluorophores (three or four) were excited using the 405, 488, 568 and 633 nm excitation-lines. The acquisition was performed using the Leica Application v3.0 or LAS X v1.0 software (Leica) or Zeiss LSM software Zen 2 Blue.

### Example 1.18. RNA isolation and RT-qPCR

Total RNA was isolated from snap-frozen muscles or cells using miRNAeasy Mini Kit (Qiagen, #1038703) and analyzed by RT-qPCR. For qPCR experiments, DNase digestion of 10 mg of RNA was performed using 2 U DNase (Qiagen, #1010395). Complementary DNA (cDNA) was synthesized from total RNA using the SuperScript^{™} III Reverse Transcriptase (Invitrogen, #18080-044). Real-time PCR reactions were performed on a LightCycler 480 System using Light Cycler 480 SYBR Green I Master reaction mix (Roche Diagnostic Corporation, #12767000) and specific primers. Thermocycling conditions were as follows: an initial step of 10 min at 95 °C, then 50 cycles of 15 s denaturation at 94 °C, 10 s annealing at 60 °C, and 15 s extension at 72 °C. Reactions were run in triplicate, and automatically detected threshold cycle values were compared between samples. Transcript of the Rpl7 housekeeping gene was used as endogenous control, with each unknown sample normalized to Rpl7 content (see **Table 2** for primers used in this study).

| **Gene** | **Forward primer** | **Reverse primer** |
|---|---|---|
| p16^{INKA} | 5' -CATCTGGAGCAGCATGGAGTC-3' SEQ ID NO: 5 | 5′-ATCATCATCACCTGAATCGGGG-3' SEQ ID NO: 6 |
| mRFP | 5' -GACCTCGGCGTCGT AGTG-3' SEQ ID NO: 7 | 5' -AAGGGCGAGATCAAGATGAG-3' SEQ ID NO: 8 |
| p19^{ARF} | 5' - TGAGGCT AGAGAGGATCTTGAGA-3' SEQ ID NO: 9 | 5' -GCAGAAGAGCTGCT ACGTGAA-3' SEQ ID NO: 10 |
| p21 | 5' -CCAGGCCAAGA TGGTGTCTT -3' SEQ ID NO: 11 | 5' -TGAGAAAGGATC AGCC A TTGC-3' SEQ ID NO: 12 |
| Il-6 | 5' -GGTGACAACCACGGCCTTCCC-3' SEQ ID NO: 13 | 5' -AAGCCTCCGACTTGTGAAGTGGT -3' SEQ ID NO: 14 |
| Il-1b | 5' -CCAAAA T ACCTGTGGCCTTGG-3' SEQ ID NO: 15 | 5' -GCTTGTGCTCTGCTTGTGAG-3' SEQ ID NO: 16 |
| PAI-1 | 5' -CCGA TGGGCTCGAGT A TGA-3' SEQ ID NO: 17 | 5' -TTGTCTGATGAGTTC AGC A TCCA-3' SEQ ID NO: 18 |
| TNFα | 5'-CGCTCTTCTGTCTACTGAACTT-3' SEQ ID NO: 19 | 5' -GATGAGAGGGAGGCCATT -3' SEQ ID NO: 20 |
| IFNγ | 5' -AGCGGCTGACTGAACTCAGATTGT AG-3' SEQ ID NO: 21 | 5' -GTCACAGTTTTCAGCTGT AT AGGG-3' SEQ ID NO: 22 |
| Il-12 | 5' - TCCAGCGCAAGAAAGAAAA-3' SEQ ID NO: 23 | 5' -AA T AGCGA TCCTGAGCTTGC-3' SEQ ID NO: 24 |
| Ccl12 | 5' -CCCACTCACCTGCTGCT ACT -3' SEQ ID NO: 25 | 5' - TCTGGACCCA TTCCTTCTTG-3' SEQ ID NO: 26 |
| Il-18 | 5' -CTGGCTGTGACCCTCTCTGT -3' SEQ ID NO: 27 | 5' -ATCTTCCTTTTGGCAAGCAA-3' SEQ ID NO: 28 |
| L7 | 5' -GAAGCTCATCT ATGAGAAGGC-3' SEQ ID NO: 29 | 5' -AAGACGAAGGAGCTGCAGAAC-3' SEQ ID NO: 30 |

### Example 1.19. Genotyping of mice

For PCR genotyping the following primers were used:
p16-3MR-1: 5'-AACGCAAACGCATGATCACTG-3' (SEQ ID NO: 31) and
p16-3MR-2: 5'-TCAGGGATGATGCATCTAGC-3' (SEQ ID NO: 32)

Positive animals show a band at 202 bp.

### Example 1.20. RNA-seq sample and library preparation

Sequencing libraries were prepared directly from the lysed cells, without a previous RNA extraction step. RNA reverse transcription and cDNA amplification were held using the SMART-Seq v4 Ultra Low Input RNA Kit for Sequencing from Clontech Takara. The Illumina "Nextera XT" kit is used for the preparation of the libraries from the amplified cDNA. Libraries were sequenced by the Illumina HiSeq 2500 sequencer (51 bp read length, single-end, ~20M reads).

### Example 1.21. Bulk RNA-seq Data Pre-processing

Sequencing reads were pre-processed employing the nf-core/rnaseq 1.2 pipeline. Read quality was assessed by FastQC 0.11.8. Trim Galore 0.5.0 was used to trim sequencing reads, eliminating Illumina adaptor remains, and to discard reads that were shorter than 20 bp. The resulting reads were mapped onto the mouse genome (GRCm38, release 81) using HiSAT2 2.1.0 and quantified using featureCounts 1.6.2. Reads per kilobase per million mapped reads (RPKM) and transcripts per million (TPM) gene expression values were calculated from the trimmed mean of M-values (TMM)-normalized counts per million (CPM) values using Bioconductor package edgeR 3.30.0 and R 4.0.0. Differential gene expression analysis and principal component analysis (PCA) were performed using Bioconductor package DESeq2 1.28.1. Variance- stabilizing transformation of count data was applied to visualize the sample-to-sample distances in PCA. Genes were considered as differentially expressed if showed adjusted p-value < 0.05.

### Example 1.22. Functional profiling of cell subpopulations

Functional enrichment analysis of the subsets of differentially expressed genes was performed using g:Profiler web server with g:SCS significance threshold, "Only annotated" statistical domain scope and canonical pathway KEGG, Reactome and Wiki Pathways sets. For each gene subset, the top five significant gene sets were selected for representation.

### Example 1.23. Gene Set Enrichment Analysis (GSEA)

RPKM matrix after removal of low count genes (edgeR 3.30.0) served as an input for GSEA 4.0.3 software. We used signal-to-noise metric to rank the genes, 1000 permutations with the gene set permutation type, and weighted enrichment statistics. Gene set sizes were chosen as 15-500 for MSigDB 7.0 Gene Ontology biological processes (GO:BP) and 10-1000 for MSigDB 7.0 canonical pathways (BioCarta, KEGG, PID, Reactome, and WikiPathways). Gene sets passing FDR < 0.25 threshold were subjected for further analysis. Network representation and clustering of GSEA results were performed using EnrichmentMap 3.2.1 and AutoAnnotate 1.3.2 for Cytoscape 3.7.2 with the Jaccard coefficient set to 0.25.

### Example 1.24. Functional profiling of SASP

We checked whether upregulated genes (DESeq2 padj < 0.05 and log2FoldChange > 0) from each Sen_vs_NSen comparison can be expressed in a form of secreted proteins by combining the evidence from multiple data sources: Gene Ontology cellular component (GO:CC), Uniprot, VerSeDa, Human Protein Atlas and experimental data reporting SASP (Freund, 2010 and SASP Atlas). The genes, which occur to be extracellular (GO:CC) and secreted (other sources) with evidence from at least 1 source were included in the final list of SASP genes (1912 in total). Functional enrichment analysis was performed using g:Profiler web server with g:SCS significance threshold, "Only annotated" statistical domain scope, and canonical pathway sets from KEGG, Reactome, and Wiki Pathways. Gene sets passing FDR < 0.25 threshold were subjected for further analysis. Network representation and clustering of g:Profiler results was performed using EnrichmentMap 3.2.1 and AutoAnnotate 1.3.2 for Cytoscape 3.7.2 with the Jaccard coefficient set to 0.25.

### Example 1.25. Comparative enrichment analysis of senescent cells and previously published aging datasets

We used the minimum hypergeometric test implemented in R package mHG 1.1 for the comparative enrichment analysis of senescent cells and previously published aging datasets: mouse (Benayoun et al., 2019), rat (GSE53960), African turquoise killifish (GSE69122), and human (GTEx v6p). Data processing and analysis were performed as described before (Brunet et al., 2019) with the following modifications: (1) to assess the differential expression for rat, killifish, and human datasets the DESeq2 1.28.1 package version was used (instead of DESeq2 1.6.3), (2) both genders were analyzed for human dataset, (3) conversion to human orthologs was performed before the enrichment with MSigDB 5.1 hallmarks for all datasets except the Brunet's mouse tissues, for which we took previously published enrichment results (Benayoun et al., 2019).

### Example 1.26. ATAC-seq sample and library preparation

ATAC-seq was performed described as Omni-ATAC-seq (Corces et al., 2017) except as described below. Sorted cells were centrifuged at 500 × g for 5min at 4°C and lysed using cold ATAC-RSB (10mM Tris- HCl, pH7.4,10mM NaCl, 3mM MgC12 supplemented in 0.1% NP40, 0.1% Tween20 and 0.1mg/ml Digitonin) on ice for 3min. Next, the lysed cells were added in 50µl of transposition mix (25 µl of 2 × TD buffer (20mM Tris-HCl, pH7.6, 10mM MgC12, 20% Dimethyl Formamide), 1.5 µl transposase (in house, (Picelli et al., 2014)), 17.5µl of PBS, 0.5µl of 10% Digitonin, 0.5µl of 10% Tween20 and 5µl of nuclease- free water). The transposition reaction was carried out for 30 min at 37°C and placed at 10°C. The transposition sample was purified using a Qiagen MinElute kit and eluted using 21µl of EB buffer. Following purification, the transposed fragments were amplified using 50ul of PCR mix (20µl of DNA, 2.5µl of custom Nextera PCR primers 1 and 2, and 25µl of KAPA HiFi HS Ready Mix for a total of 15 cycles. The PCR amplification conditions were 72°C for 5 min and 95°C for 30 sec, followed by 15 cycles at 95 °C for 10 s, 63 °C for 30 s, and 72 °C for 60 s, with a final extension at 72 °C for 5 min. Following PCR amplification, the libraries were purified, and the size was selected from 150 to 800bp using AMPure XP beads. Paired-end sequencing was performed with 50 cycles on an Illumina NovaSeq6000 platform.

### Example 1.27. Bulk ATAC-seq Data Pre-processing

Read quality was assessed by FastQC 0.11.8. All adapters were removed by Fastp 0.21.0. The clean data was then aligned to mm10 mouse genome assembly using Bowtie2 2.2.5 with the settings "--very sensitive". Low mapping quality reads were removed using samtools 1.3.1 with the settings "-q 30". BigWig files were generated using deeptools 3.3.1 with the settings "-normalizeUsing CPM". Peaks were called using Macs2 2.1.0 with the options "--nomodel - -keep-dup -q 0.01". For differential accessibility analysis, union peak sets were created by Bedtools 2.29.2, reads corresponding to each region were assigned by FeatureCounts. Differentially accessible peaks were identified using DESeq2 1.24.0 with the criteria of adjusted p-value < 0.1 and an absolute value of log2FoldChange > 1. Differentially accessible peaks were further annotated by HOMER 4.10.4, the associated motif enrichment analysis was performed by HOMER with default settings.

### Example 1.28. Analysis of senescence-induced changes in promoter chromatin accessibility

An MA-plot (log2 fold change vs. mean average) was used to visualize changes in chromatin accessibility for all peaks. As a peak score we used an average of TPM-normalized read counts: (1) reads per kilobase were calculated by division of read counts by the length of each peak in kilobases; (2) "per million" scaling factor was calculated as a sum of all reads per kilobase for each sample; (3) reads per kilobase were divided by "per million" scaling factor; (4) peaks with "promoter-TSS" annotation (TSS^{+/-}1kb) were selected and the average was calculated for each group. For MA-plots, we only included those peaks with average normalized signal > 5. The number of peaks with log2 fold change >1 or <-1 was calculated as well as the density of the peaks with negative log2 fold change. Normalized ATAC-seq signal profiles of proximal promoters were visualized for key genes using Integrative Genomic Viewer v.2.8.13.

### Example 1.29. Transcription factor analysis and activity prediction

For the analysis of transcription regulation we combined the results of several methods: (1) motif enrichment analysis of differentially expressed genes with TRANSFAC_and_JASPAR_PWMs and ENCODE_and_ChEA_Consensus_TFs_from_ChIP-X libraries using R package EnrichR 2.1; (2) Upstream Regulator Analysis of differentially expressed genes using the commercial QIAGEN's Ingenuity Pathway Analysis (IPA, QIAGEN Aarhus, Denmark) software; (3) analysis of transcription regulators differential expression using DESeq2 1.28.1; (4) motif enrichment analysis of differentially accessible regions using HOMER 4.10.4.

Potential regulators from EnrichR and IPA results passing the threshold of p-value < 0.05 were used to build a union set of transcription regulators, which was further filtered to retain only the molecules with DESeq2 baseMean value > 0. For further validation of the activity status transcription regulators were matched to the known HOMER motifs passing the Benjamini q-value < 0.05 threshold.

A discrete scoring scale (Inhibited, Possibly inhibited, Unknown/Contradictory, Possibly activated, Activated) was used to evaluate the activity of transcription regulators based on combined evidence from EnrichR, IPA, DESeq2, and HOMER results. We used z-score statistics to define the activity status of transcription regulators from EnrichR analysis results by matching the differential expression of target genes with activatory and inhibitory interactions from the Bioconductor package DoRothEA 1.0.0 and web- based TRRUST v2 database. Activity prediction was adjusted by the overlap of matched interactions with the total number of target genes. To define the activity status of transcription regulator from IPA Upstream Regulators Analysis results, IPA-calculated z-score and analysis bias were taken into account. Activity predictions were further corrected by differential expression of transcription regulators using DESeq2. Expression z-score statistical value was calculated to functionally classify transcription regulators as activators or repressors based on the proportion of upregulated and downregulated target genes. We further calculated chromatin accessibility z-score to estimate the prevalence of HOMER motif enrichment in open versus closed regions that together with transcription regulators function allowed us to predict their activities based on ATAC-seq data and use it for validation of activity predictions based on RNA-seq results.

To estimate the level of confidence, for each enrichment result we calculated a discrete "Trust" score, with each point assigned for: (1) EnrichR p-adjusted < 0.05; (2) IPA p-value < 0.05; (3) activity status "Activated" or "Inhibited"; (4) unidirectional |z-scores| > 2 from both EnrichR and IPA results; (5) concordance between the differential expression of the transcription regulator and the prediction of its activity score; (6) activity validated by the analysis of ATAC-seq data. Transcription regulators with average Trust > 1 were subjected to further analysis.

### Example 1.30. Functional profiling of transcription factor target gene regulation

For each transcription factor, we merged the target genes from EnrichR and IPA results split them into upregulated and downregulated, and subjected them to functional enrichment analysis of canonical pathways (KEGG, Reactome) and GO:BP using R package gprofiler2 0.1.9 with the following parameters: correction method "FDR", "custom_annotated" domain score consisting of target genes for all studied transcription factors. Electronic GO annotations were excluded. Gene sets passing FDR < 0.05 threshold were subjected for further analysis. For GO:BP we selected the ones having term size > 15 and < 500 genes. For functional profiling, we took the clusters of gene sets created using AutoAnnotate Cytoscape App based on GSEA results. We retained all the terms present in these clusters and extended the lists with terms from gprofiler2 enrichment results, adding them by semantic and functional similarity. As result we had a library of terms grouped by clusters, which allowed us to match them with gprofiler2 results and, thus, to map transcription factors to main functional clusters from GSEA/Cytoscape analysis.

Transcription factors mapped to the same functional cluster in ≥ 8 (out of 12) Sen_vs_NSen comparisons were subjected to further filtering. We scored as 1 point in each case when any of the following attributes had a value above the upper quantile for a given cluster: number of comparisons, % of GSEA terms among all terms with enrichment, -log10 of the average minimum FDR, average Trust score. Additionally, we scored as 1 point if the transcription factor was associated with senescence in literature. For graphical representation, we selected examples of transcription factors and target genes based on literature research: 19 transcription factors (out of 29 with the score ≥ 2) mapped to 9 clusters ("Matrix remodeling/fibrosis", "Interferon signaling", "Chemotaxis", "Lipid uptake", "IGF regulation", "Detoxification", "Gene expression and protein translation", "Cell cycle", "DNA repair").

### Example 1.31. Functional profiling of transcriptional regulation of SASP

For each transcription factor upregulated target genes from EnrichR and IPA results were merged and intersected with the list of SASP genes. For SASP genes we extracted Gene Ontology molecular function (GO:MF) terms, clustered them into 12 categories ("Adhesion molecule", "Chemokine", "Complement component", "Cytokine", "Enzyme", "Enzyme regulator", "Extracellular matrix constituent", "Growth factor", "Hormone", "Ligand", "Proteinase" and "Receptor) and estimated the enrichment of GO:MF clusters with a hypergeometric test using R function "phyper". Correction for multiple comparisons was performed using the Benjamini-Hochberg procedure.

Transcription factors having enrichment of the same GO:MF cluster with p-value < 0.05 in ≥ 8 (out of 12) Sen_vs_NSen comparisons were subjected to further filtering. We scored as 1 point in each case when any of the following attributes had a value above the upper quantile for a given cluster: % of secreted proteins among targets, number of comparisons with p-value < 0.05, number of comparisons with p-adjusted < 0.05,
-log10 of the average p-value, average Trust score. Additionally, we used ATAC-seq data analysis to score as 1 point the presence of transcription factor motif in the promoter region of at least one SASP gene within the cluster. For graphical representation, we selected 17 transcription factors with score ≥ 2 and with ≥ 3 comparisons with p-adjusted < 0.05. They were associated with 5 GO:MF categories ("Extracellular matrix constituent", "Cytokine", "Chemokine", "Complement component", and "Growth factor"), for which we selected the most common target genes.

### Example 1.32. Analysis of lipid metabolism gene set

For the analysis of lipid metabolism, we constructed a gene set using data from multiple sources: KEGG pathway maps ("Fatty acid degradation", "Cholesterol metabolism", "Regulation of lipolysis in adipocytes"), WikiPathways ("Fatty acid oxidation", "Fatty Acid Beta Oxidation", "Mitochondrial LC- Fatty Acid Beta-Oxidation", "Fatty Acid Omega Oxidation", "Fatty Acid Biosynthesis", "Triacylglyceride Synthesis", "Sphingolipid Metabolism (general overview)", "Sphingolipid Metabolism (integrated pathway)", "Cholesterol metabolism (includes both Bloch and Kandutsch-Russell pathways)", "Cholesterol Biosynthesis"), literature research (Flor et al., 2017; Stahl, 2004; Zani et al., 2015). We further estimated the expression of these genes by filtering DESeq2 results (padj < 0.05 in at least 3 out of 12 comparisons) and extracted log2FoldChange values to plot the difference in expression between senescent and non- senescent cells.

### Example 1.33. Reconstruction of ligand-receptor mediated cell-cell communication networks

For reconstructing cell-cell communication networks, we modified a previously published single-cell based method, FunRes, to account for bulk gene expression profiles (Jung et al., 2020). In brief, the method consists of four steps. First, TFs, ligands, and receptors expressed across all replicates are detected for each cell population. Here, TFs having an expression value of more than 1 TPM are considered expressed.

Second, receptors regulating these TFs are detected by employing a Markov Chain model of signal transduction to detect high-probability intermediate signaling molecules (Ravichandran et al., 2019). These molecules are connected with receptors based on a signaling interaction network. Subsequently, all shortest paths from receptors to TFs are determined and scored based on the expression of genes along these paths and the mode of action of their constituent interactions. Significance is assessed by calculating the z-score of each path over random receptor-TF associations. Z-scores above 2 were considered significant. Third, ligand-receptor interactions between two cell populations are reconstructed if (i) the receptor is expressed and regulates any TF, (ii) the ligand is expressed, and (iii) the receptor-ligand interaction is contained in the cell-cell interaction scaffold. Finally, a score is assigned to every interaction by multiplying the average receptor and ligand expression in their respective cell populations. Significance is assessed by permuting cell population labels 100 times and re-computing the interaction scores in the permuted datasets. Interactions are considered significant if it is at least two standard deviations greater than the mean of the permuted interaction scores. Only significant interactions are retained in the final network.

### Example 1.34. Downstream analysis of senescence-induced ligand-receptor interactions

For the functional profiling, we selected ligand-receptor interactions between three senescent cell populations (SCs, FAPs, and Ms) and non-senescent SC population in geriatric mice 3 DPI. We used Bioconductor package SPIA 2.40.0 with a reduced set of non-disease KEGG pathways maps to evaluate the activity of pathways downstream ligand-receptor interactions. For each interaction differentially expressed interface TFs in non-senescent SC were split into upregulated and downregulated in comparison with senescent SCs. As a reference set of genes, we took a list of interface TFs from all the interactions studied. SPIA analysis was performed with 2000 permutations, pPERT and pNDE were combined with Fisher's product method. Pathways were considered as significantly enriched if passing a pGFdr < 0.05 threshold. For each pathway, we calculated the ratio of ligand-receptor interactions that activate or inhibit the pathway to the total number of interactions analyzed. For results representation, we selected 8 activated and 8 inhibited pathways with the highest ratio of interactions.

### Example 1.35. Statistical analysis

The sample size of each experimental group is described in the corresponding figure caption, and all the experiments were conducted with at least three biological replicates unless otherwise indicated. GraphPad Prism software was used for all statistical analyses except for sequencing-data analysis. Quantitative data displayed as histograms are expressed as mean ± standard error of the mean (represented as error bars). Results from each group were averaged and used to calculate descriptive statistics. Mann-Whitney test (independent samples, two-tailed) was used for comparisons between groups unless otherwise indicated. Statistical significance was set at a P-value <0.05.

### Example 2. Results

### Example 2.1. The muscle regenerative niche acquires senescent cells after injury

To study cellular senescence in skeletal muscle, we used p16-3MR mice, which express Renilla luciferase, RFP (monomeric red fluorescent protein), and a viral thymidine kinase under the p16^{INK4a} promoter (Demaria et al., 2014). We induced injury by intramuscular injection of cardiotoxin (CTX) into either young mice (aged 3-6 months), which have very efficient regeneration, or geriatric (very old) mice (over 28 months), which show maximal regenerative failure (Garcia-Prat et al., 2016; Sousa-Victor et al., 2014). Luciferase activity (a proxy of p16^{INK4a}-expressing senescent cells) was undetectable in resting muscles of young mice but was sharply induced at 3 days post-injury (DPI) and was still elevated at 7 DPI, after which it decreased (**Figures 1A**). In p16-3MR mice, senescent cell detection relies on p16^{INK4a} transgene expression; we therefore tracked senescence-associated beta-galactosidase (SA-β-gal) activity as a senescent-cell marker in muscles of wild-type (WT) mice. Senescent cells were present only in damaged muscles, with their number peaking at day 3 post-injury and decreasing thereafter in young WT mice. Senescent cells were more abundant and persisted longer in injured muscles from geriatric mice (**Figure 1B**), correlating with their less efficient recovery process. Analysis of additional markers confirmed that injury induced the transient accumulation of senescent cells in both age groups.

Niche constituents in regenerating tissues remain largely undefined. Immunostaining of senescence markers (p16^{INK4a} and γH2A) labeled cells close to known niche constituents, such as SCs (using Pax7⁺), FAPs (using TCF4⁺), and Mϕs (using CD11b⁺). Furthermore, markers for SCs, FAPs, and Mϕs co- localized with senescent cell markers (**Figure 1C**), suggesting that senescent cells emerge from and may influence normal niche constituents after injury.

As in mouse muscle, SA-β-gal and p16^{INK4a} were present in biopsies of regenerating adult human muscle but not intact adult human muscle (**Figure 1D**). Furthermore, regenerating human muscle contained SCs (using NCAM/CD56⁺), FAPs (using PDGFRa⁺), and Mϕs (using CD68⁺) that were positive for 53BP1 (**Figure 1E**). Thus, senescent cells are specifically induced in damaged muscles of both humans and mice.

To determine the nature of these senescent cells, we established a fluorescence-activated cell sorting (FACS) method based on the coupling of cell-surface markers of SCs, FAPs and Mϕs to a SPiDER-β-gal reagent (Doura et al., 2016), which labels all SA-β-gal⁺ cells and does not rely on p16^{INK4a}-dependent reporter expression (Demaria et al., 2014). No SPiDER⁺ cells were detected in unperturbed muscle. However, in regenerating muscles, SPiDER⁺, and SPiDER⁻ subpopulations of SCs, FAPs, and Mϕs could be simultaneously isolated and validated by immunostaining and gene expression. An additional fraction of CD45⁺/F480⁻ SPiDER⁺ cells was also observed, indicating that immune cells (including Mϕs) were the predominant senescent-cell subpopulation. Senescence was validated in the SPiDER⁺ subpopulations (from hereon, senescent (Sen) cells) by increased SA-β-gal activity, large cell size, lack of proliferation, and decreased lamin B1 expression compared with their SPiDER⁻ non-senescent (NSen) cell counterparts (**Figures 1F** **and** **1G**). This confirmed the identity of new senescent niche constituents after tissue injury and provided a novel strategy for isolating distinct senescent cell types *in vivo.*

### Example 2.2. Cellular senescence represses muscle regeneration throughout life

We next studied the role of senescent cells in young and geriatric mice during regeneration. We first ablated p16^{INK4a}-expressing senescent cells specifically by daily administration of ganciclovir (GCV) to young and geriatric p16-3MR mice. In both groups, GCV reduced the presence of senescent cells in injured muscles, indexed by lower luciferase activity *in vivo* and reduced SA-β-gal⁺ cells. In geriatric p16-3MR mice, GCV not only rescued the defective regeneration (**Figure 2A**) but also enhanced muscle force generation (**Figure 2B**). Unexpectedly, these beneficial effects were also evident in GCV-treated young mice (**Figure 2B**) and were accompanied by reduced inflammation and fibrosis in both young and geriatric mice.

As an alternative with potential application in humans, we explored the effects of distinct senolytic compounds that selectively induce apoptosis (Kirkland and Tchkonia, 2020). We co- administered quercetin and dasatinib (Q⁺D) (Xu et al., 2018), the most effective senolytics that deplete senescent cells. Similar to GCV treatment, daily Q⁺D administration reduced SA-β-gal⁺ cell numbers, accelerated regeneration, increased force, and reduced inflammation and matrix deposition, in injured muscles of both young and geriatric WT mice (**Figures 2C**). Similar benefits were obtained when GCV or senolytics were transiently administered (for four days starting at 3 DPI) to young and geriatric mice. Thus, elimination of senescent cells reduces the inflammatory and fibrotic load in the regenerative niche, accelerates regeneration in young mice, and rejuvenates muscles of extremely old mice that are usually refractory to any improvement.

Consistently, transplantation of Dil-labelled sorted Sen (SPiDER⁺) or NSen (SPiDER⁻) cells into pre- injured muscles of young mice revealed that only Sen cells delayed the regeneration of host muscles (**Figure 2D**), mimicking the defective regeneration in geriatric muscles. Thus, senescent cells are detrimental to muscle regeneration in both young and aged mice. These findings challenge the prevalent view that senescent cells are beneficial when present transiently after acute injury, particularly in young tissues (revised in (Prieto et al., 2020; Rhinn et al., 2019)).

### Example 2.3. Transient or persistent accumulation of senescent cells is detrimental to muscle regeneration

To exclude the possibility that the observed roles of senescent cells depend on the degree of tissue injury, we 1) induced transient, mild injury with localized muscle micropunctures (Desguerre et al., 2012) and 2) examined the chronic, severe injury naturally occurring in mdx mice, a model of Duchenne muscular dystrophy (DMD) (Serrano and Munoz-Canoves, 2017). Muscle micropunctures induced acute and transient SA-β-gal⁺ cells and luminescence activity in the lesioned area of WT and p16-3MR mice, respectively (**Figure 2E**). In these models, senescent cell elimination in p16-3MR mice (by GCV) or in WT mice (by Q⁺D) enhanced regenerating myofiber growth and reduced inflammation and matrix deposition (**Figure 2F**), confirming that even the transient presence of low numbers of senescent cells is prejudicial for the repair of mildly damaged muscles.

We next brought p16-3MR mice to the mdx dystrophic background (characterized by chronic muscle degeneration/regeneration cycles). Monitoring of luciferase activity and additional senescence markers showed that senescent cells were more numerous in the muscles of mdx/p16-3MR mice than in age- matched, non-dystrophic p16-3MR mice, and were detectable for months (**Figure 2G**). This is in agreement with the chronic injury to mdx muscles. Importantly, treating mdx/p16-3MR mice with GCV and mdx mice with Q⁺D senolytics, twice weekly for 2 months, reduced the senescent-cell burden and alleviated the regenerative impairment of dystrophic muscles, as shown by larger fibers, reduced fibrosis, and enhanced muscle force (**Figures 2H** and **2I**). These results demonstrated that the irruption of senescent cells in the regenerative muscle niche, either transiently (in mild injury) or persistently (in chronic injury), is always deleterious for regeneration, irrespective of age.

### Example 2.4. Senescent cells retain their homeostatic identity while gaining lineage-inappropriate functions in old age

Transcriptomics of senescent cells has previously been only performed *in vitro* due to the inability to separate them alive (Basisty et al., 2020; Childs et al., 2015; Hernandez-Segura et al., 2017). From young and geriatric mice, we purified muscle Sen (SPiDER⁺) and NSen (SPiDER⁻) subpopulations of SCs, FAPs and Mϕs at early (3 DPI) and late (7 DPI) phases of regeneration and NSen (SPiDER⁻) cells from uninjured muscles (Basal) of the contralateral legs. This inclusion of 36 distinct *in vivo* conditions (combinations of 3 distinct cell types, 3 cell states, 2 regeneration timepoints, and 2 animal ages) is a key strength of our datasets, in stark contrast to all prior studies, in which analysis was confined to senescent cells *in vitro.*

RNA-sequencing (RNA-seq)-coupled bioinformatics analysis and principal component analysis (PCA) of all datasets revealed that transcriptomes primarily clustered by cell type, rather than cell state, regeneration timepoint or animal age (**Figure 3A**). However, Sen SCs and Mϕs from geriatric mice at 3 DPI were transcriptionally closer to each other than to their NSen or Basal counterparts (**Figure 3A**). Within a particular cell type, there was clear segregation according to the cell state, with a strong influence of senescence.

Comparison between Sen and NSen subpopulations at 3 DPI in young mice showed 4958 differentially expressed (DE) genes in Sen Mcps, 2251 in Sen SCs, and 1805 in Sen FAPs, indicating high transcriptional heterogeneity of senescent cells even within the same niche. Basic cell processes, such as transcription, RNA metabolism and translation, were downregulated in all three Sen subpopulations (**Figure 3B**); nevertheless, many DE genes and associated enriched pathways were specific to each Sen cell type and revealed the cell of origin (**Figure 3B**). For instance, young Sen SCs expressed genes related to muscle contraction and integrin/cell surface interactions, reminiscent of myogenic homeostatic functions. In contrast, Sen FAPs expressed genes related to the actin cytoskeleton and elastic fiber regulation, suggesting an attempt of these matrix cells to regulate their shape to occupy a larger space within the damaged muscle area, and Sen Mϕs expressed genes related to innate immune functions and high lysosomal content, which are associated to myeloid cell homeostatic functions (**Figure 3B**). This cell-of- origin memory accounts in part for the heterogeneity of senescent cells within the regenerative niche. With aging, senescent cells gained alternative cell-fate traits: geriatric Sen FAPs showed a pro-inflammatory profile, with upregulated pathways of macrophage markers and complement and coagulation cascades (**Figure 3C**)**;** geriatric Sen Mϕs acquired functions related to ECM organization and collagen formation (**Figure 3D**); and geriatric Sen SCs upregulated immune system functions, such as chemokine signaling pathway, immune system, and osteoclast differentiation, and downregulated cell cycle and DNA repair pathways (**Figure 3E**). Furthermore, all geriatric Sen cells gained additional cell plasticity functions, including patterning and development (e.g., post-embryonic animal organ development, regulation of heart morphogenesis, and respiratory system development) and had exacerbated pro-inflammatory traits (such as the cellular response to prostaglandin, regulation of macrophage chemotaxis, and lipid transport-related inflammatory pathways) (**Figure 3F**). These results indicate that senescent cell heterogeneity after tissue injury results from the maintenance of particular identity and cell homeostatic functions, as well as acquisition of non-self traits, in old age. Thus, in addition to the higher number of senescent cells with age (**Figure 1B**), aged Sen cells also have exacerbated inflammatory functions, and these features together may contribute to the regenerative failure of muscles in extreme old age.

### Example 2.5. Tissue injury drives senescence by inducing severe oxidative stress and DNA damage in a subset of niche cells

To understand how senescence is induced in the regenerative niche, we searched for pathways enriched in Sen cells early after muscle injury (3 DPI). Pathways related to DNA damage, cell-cycle checkpoints, oxidative phosphorylation, mitochondrial biogenesis, and some inflammatory pathways were upregulated, whereas pathways related to homeostatic gene-expression control and protein translation were downregulated (**Figure 4A**). Compared with proliferating NSen cells from injured muscles, Sen cells were enriched in pathways more specifically implicated in cellular stress, such as oxidative and metabolic stress (including ROS and OXPHOS production and lipid transport and metabolism), with concomitant downregulation of pathways implicated in DNA damage repair responses and mitochondrial functions (**Figure 4B**). We thus hypothesized that high levels of DNA damage and high oxidative stress caused by injury are not efficiently repaired in a fraction of niche cells, triggering them to enter senescence rather than their normal proliferative fate. Supporting this idea, all freshly-sorted Sen cell types (SCs, FAPs, and Mϕs) had more DNA damage foci than NSen or Basal cells (as shown by γH2Ax immunostaining) (**Figure 4C**) and produced more ROS, which may be related to mitochondrial dysfunction in Sen cells (**Figure 4B**). Further, Sen SCs, FAPs, and Mϕs at 3 DPI had more intense CellRox (ROS) staining than their NSen (or Basal) counterparts (**Figure 4C**). These results further validate the SPiDER-based FACS detection of distinct senescent cell types *in vivo.*

To assess the causality of high ROS levels in driving injury-induced senescence, we sorted ROS^{High} and ROS^{Low} SCs and FAPs at 1 DPI (before the appearance of fully senescent cells at 3 DPI). Freshly-sorted ROS^{High} cells, but not ROS^{Low} cells, readily became senescent in culture (shown by SA-β-gal staining) (**Figure 4D**). The senescence burden was reduced by inhibiting ROS with the antioxidant drug N- acetylcysteine (NAC) during regeneration, as shown by Renilla luciferase activity *in vivo* and SA-β-gal staining in tissue (**Figure 4E**). Similar results were obtained with *in vitro* studies, in which NAC blocked senescence entry of ROS^{High} cells (**Figure 4F**). Interestingly, Sen cells showed specifically altered lipid transport and lipoprotein remodeling functions (**Figure 4B**), consistent with the capacity of oxidative stress to generate a lipotoxic environment that triggers inflammation and fibrosis progression (Wree et al., 2014).

### Example 2.6. Aging primes niche cells for exacerbated injury-induced senescence

To investigate the causes of exacerbated senescence in geriatric cells, we analyzed the sole effect of aging on resting cells in unperturbed muscles. Comparison of geriatric and young Basal cells from the three lineages revealed that aging upregulated functions associated with the immune-inflammatory response, DNA damage and cell cycle arrest, lipid metabolism, matrix remodeling, and insulin signaling, and downregulated pathways associated with mitochondrial maintenance and function (**Figure 4G**). Thus, in resting tissue, aging alters cellular functions that are similar to those altered by tissue injury. Accordingly, geriatric Basal cells contained more DNA damage foci than young Basal cells, as shown by telomere immunostaining of γH2Ax (**Figure 4H**). This coincided with previously reported increased ROS levels in geriatric cells (Garcia-Prat et al., 2016), suggesting that the intrinsic accumulation of damage primes aged cells for senescence entry upon injury. We also confirmed more prominent matrix deposition (**Figure 4H**) and expression of cell-cycle inhibitors and inflammatory factors in resting geriatric than in young muscles (**Figure 4I**), which is consistent with the concept of basal age-associated inflammation (inflammaging) (Franceschi and Campisi, 2014). Together, our results show that subsets of cells within the muscle regenerative niche of young (and old) mice accumulate very high levels of oxidative stress and DNA damage in response to injury and become enriched in abnormal lipid transport and mitochondrial pathways, all of which drive senescence entry. Aging also leads to the accumulation of intracellular stressors and activation of inflammatory-response pathways, thus priming niche cells for senescence, which in turn results in a deeper senescent state upon injury.

### Example 2.7. Two major shared hallmarks define senescent cells across cell types, stages of regeneration and lifespan

We next searched for commonly regulated traits in Sen cells across all conditions. Despite the limited coincidence at the gene-expression level, pathway enrichment analysis of DE genes between Sen and NSen cells for each cell type in any given condition showed upregulation of two major global functions: inflammation (complement and coagulation, chemotaxis, interferon signaling, and lipid uptake by scavenger receptors) and matrix remodeling/fibrosis (ECM glycoproteins and cell adhesion) (**Figure 5A**). Minor shared conserved functions were related to stress responses (cell detoxification and transport of small molecules) and IGF regulation (regulation of IGF transport and uptake by IGFBPs) (**Figure 5A**). Conversely, the basic cellular machinery (including gene expression to protein translation, cell cycle and DNA repair) (**Figure 5A**) was strongly downregulated across all conditions. Thus, rather than having closely aligned changes in the expression of a fixed set of genes, Sen cells have a common profile of cellular pathways, establishing core senescence hallmarks shared across all cell types, regeneration kinetics and ages. Comparison of Sen cells with their Basal counterparts showed that core senescence hallmarks were largely maintained, indicating the uniqueness of the senescent state across all conditions (**Figure 5B**) and excluding the possibility that they are due to the cell growth arrest (i.e. quiescence) state per se. To the best of our knowledge, this atlas of cellular senescence in skeletal muscle provides the first molecular and functional definition of senescent cells *in vivo* throughout life.

### Example 2.8. Transcription factor occupancy and gene accessibility reflects the activation of major inflammatory and fibrotic senescence hallmarks

To investigate the molecular specification of senescence, we performed transcription factor (TF) enrichment analysis and pathway enrichment analysis of TF targets. This allowed us to map common TFs (**Figure 5C**) to the main pathway hallmarks enriched in Sen cells in all conditions. This search revealed enrichment occupancy of known transcriptional regulators of inflammation and the SASP, NF-κB (Tilstra et al., 2012), C/EBPβ (Kuilman et al., 2008) and STAT1/3 (Kandhaya-Pillai et al., 2017), as well as the regulators of fibrosis and matrix remodeling, Smad3/4 (and inhibition of Smad7) (Meng et al., 2016) (**Figure 5D**). We next investigated changes in chromatin structure by transposase-accessible chromatin high-throughput sequencing (ATAC-seq) in Sen and NSen subpopulations of FAPs, Mϕs and SCs at early and late phases of regeneration (and in Basal NSen cells) from young and geriatric mice. This analysis revealed the accessibility of senescence-defining genes in our transcriptomic data. By comparing the signal for each ATAC-seq peak in NSen and Sen cells, we observed total reduced chromatin accessibility in Sen cells (**Figure 5E**). In geriatric cells, promoters with medium peaks were more accessible. In the key genes, Ifnb1 and Ccl8, the peaks in the proximal promoter were increased in Sen cells, correlating with increased expression (**Figure 5F**). Thus, transcriptomic profiling and chromatin accessibility identification of senescence hallmarks in vivo reinforced the inflammatory/fibrotic-like nature of senescent cells within the muscle niche, regardless of age or time after injury.

### Example 2.9. Senescent cells create an aged-like microenvironment in young regenerative niches through the secretion of pro-inflammatory and pro-fibrotic factors

To understand how senescent cells impair regeneration, we studied their SASP in vivo, in regenerating muscles, and during aging. We first selected DE genes with extracellular or secreted protein products and compared them between injury-induced Sen versus NSen cells. Depending on cell type and conditions, the number of SASP components ranged from 78 to 365, highlighting the diversity in SASP production, in line with the gene expression heterogeneity of senescent cells. Pathway enrichment of the SASP genes identified two major common functions: 1) a pro-inflammatory function, including complement and coagulation, innate immune system, lipoprotein remodeling, and cytokines and TNFα/NEκB signaling; and 2) a pro-fibrotic function, including matrix organization and collagen metabolism, matrix metalloproteinases and TGFβ signaling. Other functions, such as IGF regulation by IGFBPs (**Figure 6A**), were also present. Overall, these results indicated that major SASP functions correspond tightly with the universal hallmarks of senescent cells in vivo (**Figure 5A**).

Increased basal inflammation of aged tissues and organs (inflammaging) is a hallmark of aging (Franceschi and Campisi, 2014). Prompted by the marked inflammatory character of senescent cells, we compared the transcriptomes of aging tissues from various species (including human) with those of the distinct Sen cells in injured muscles (particularly those of young muscle). Our transcriptomic SASP data sets displayed an increase in pathways related to inflammatory function, including interferon, complement, and cytokine signaling, or TNFα/NFκB signaling (**Figure 6B**), consistent with the wealth of published transcriptional aging datasets of human, rodent, and killifish, at least at the pathways level (Benayoun et al., 2019; Stegeman and Weake, 2017). Supporting this, a secreted-protein array-based assay confirmed the effective secretion of inflammatory (and matrix-remodeling) proteins, such as Ccl2, Cxcl1, Cxcl5, IL-12, and Mmp3, in freshly-sorted Sen cells from young muscles (**Figure 6C**). Thus, the SASP of all Sen cells transiently present in injured young muscles mimics an "aged-like" inflammaging (Benayoun et al., 2019; Franceschi and Campisi, 2014; Stegeman and Weake, 2017). In geriatric muscle, the inflamed senescent-cell niche we observed after injury may further exacerbate existing inflammaging (**Figures 4H** **and 4J**). Moreover, functional profiling of the transcriptional regulation of SASP confirmed the association of NF-κB, STAT1/3, and Smad3/4 with the identified groups of inflammatory and matrix-related SASP genes (**Figure 6D**), correlating with higher accessibility (**Figure 5E** **and** **5F**), and reinforcing the transcriptional control of the inflammatory and fibrotic SASP. To assess the role of NF-κB and Smad3 TFs as possible links between senescence and the inflammatory SASP in regenerating muscles, we treated young mice after injury with either an NF-κB pathway inhibitor (Bortezomib) (Liu et al., 2017) or a Smad3 inhibitor (SIS3) (Tang et al., 2017). Both Bortezomib and SIS3 diminished the expression of pro-inflammatory and pro-fibrotic SASP factors in freshly-sorted SPiDER⁺ (Sen⁺) cells, based on secreted-protein array-based assay, and the transcription of selected SASP genes. Because NF-κB and Smad3 are associated with inflammation and aging (Chen et al., 2018; Salminen et al., 2008; Shavlakadze et al., 2019; van der Kraan et al., 2010), our results suggest that these TFs link injury-induced senescence and inflammaging *in vivo.*

### Example 2.10. Senescent cells block muscle stem-cell expansion through the SASP paracrine actions

To further assess the impact of senescent-cell SASP components on muscle regeneration (and particularly on muscle SCs), we reconstructed ligand-receptor (L-R) interactions between Sen cells (producing SASP ligands) and Nsen SCs (harboring cognate receptors) using a modified version of FunRes (Jung et al., 2020), revealing predominantly inflammatory interactions. Signaling pathway impact analysis (SPIA) (Tarca et al., 2009) of TFs acting downstream of these interactions revealed common downstream responses induced by SASP ligands on NSen SCs. The SASP produced by all Sen cells activated functions in the receiving SCs (such as cellular senescence, apoptosis and inflammatory responses, including TNFα and TGFβ signaling), and inhibited cell cycle and proliferative pathways (such as the MAPK and AKT signaling cascades). Thus, SASP components might provoke either proliferative arrest or paracrine senescence (through a bystander effect) in NSen SCs. To test these predictions, we transplanted sorted SPiDER⁺ (Sen) cells and SPiDER- (NSen) cells, labeled with the lipophilic vital dye Dil, into pre-injured muscles of recipient mice. In contrast to Dil-labelled NSen cells, Dil-labelled Sen cells increased the number of senescent cells in the host tissue, induced recruitment of inflammatory cells to the damaged area, and increased fibrosis (**Figure 6E**). Furthermore, paracrine senescence induction was confirmed by transplantation of ex vivo-induced WT senescent (or non-senescent) cells into pre-injured muscles of p16-3MR reporter mice, evidenced by enhanced luciferase activity in muscles transplanted with senescent cells (**Figure 6F**). These effects may explain the delayed regeneration of host muscles (**Figure 2D**). Conversely, loss of senescent cells in GCV-treated p16-3MR mice increased the number of proliferating SCs within the regenerative muscle niche (**Figures 6G**), in agreement with the accelerated muscle regeneration. Consistent with these in vivo results, SCs sorted from GCV- treated p16-3MR mice had a higher proliferation capacity ex vivo than SCs from vehicle-treated mice (**Figure 6G**). Furthermore, transwell assays with Sen cells reduced the proliferation rate of NSen SCs ex vivo (**Figures 6H**). Together, these findings demonstrate that senescent cells restrain muscle regeneration through paracrine pro-inflammatory and pro-fibrotic SASP functions that blunt SCs proliferation.

To examine the relative contribution to muscle regeneration of the distinct types of senescent cells (tissue- resident and blood-derived), we first used a model of whole-muscle grafting (Sousa-Victor et al., 2014), in which the extensor digitorum longus (EDL) muscle from one mouse is grafted onto the tibialis anterior (TA) muscle of a recipient. In this model, myofibers of the transplanted EDL muscle undergo de novo myogenesis at the expense of its own SCs, while recruited bone marrow-derived cells come from the host. EDL-grafting combined with GCV-mediated senescent-cell depletion revealed that p16-3MR-EDL grafts in WT host mice, or WT-EDL grafts in p16-3MR hosts, had larger regenerating myofibers than WT-EDL grafts in WT mice (**Figure 6I**). These results confirmed that EDL-resident senescent SCs and FAPs, as well as blood-derived inflammatory senescent cells, blocked muscle regeneration. Similar detrimental effects on muscle regeneration were seen upon transplantation of equal numbers of Sen (SPiDER⁺) SCs, FAPs, or Mcps, either separately or combined, independently of Sen cell type (**Figure 6J****;** **Figure 2D**). These results strongly suggest that that conserved secretory inflammatory and fibrotic functional hallmarks across the three Sen cell types account for their similar negative effects on tissue regeneration. Consistent with this idea, improved muscle regeneration after senescent-cell depletion was always accompanied by reduced inflammation and fibrosis in both young and aged muscles, whereas the reduced muscle regeneration rate caused by the paracrine action of transplanted Sen cells was associated with increased muscle inflammation and fibrosis (**Figure 6E**). These findings further challenge the prevalent view that the SASP of senescent cells is beneficial when acting transiently after injury.

### Example 2.11. CD36 neutralization improves regeneration in young and old mice through a senomorphic action

Given the heterogeneity of SASPs *in vivo,* we searched for a broad SASP-targeting approach to provide further evidence for the beneficial effects of reducing the SASP in vivo. Lipid transport function, which is tightly associated with inflammatory responses (Connelly et al., 2016; Nazir et al., 2020), was consistently included in the inflammatory hallmark of Sen cells in all conditions **(****Figure 5A**). Moreover, enrichment of SASP-related genes identified lipid transport by lipoproteins as a core SASP function **(****Figure 6A**), strongly suggesting that it might intervene in the paracrine functions of senescent cells. Senescent cells show lipid metabolism changes (Flor et al., 2017) and accumulation of lipofuscin (a commonly used marker of senescence composed of oxidized lipids and proteins (von Zglinicki et al., 1995); however, the role of lipid uptake in the context of cellular senescence remains unclear, especially in vivo.

Sen cells had more lipid droplets than NSen cells **(****Figure 7A**). Analysis of lipid metabolism genes identified upregulation in all Sen cells of numerous lipid-transport genes, including Fabp3, Apoe, Star, Pltp, Lpl, Cd68, and Cd36 **(****Figure 7B**). Lipid uptake and CD36 are related to SASP *in vitro* (Chong et al., 2018; Saitou et al., 2018). Therefore, given that Cd36 was consistently upregulated in all the Sen cell conditions **(****Figure 7B**), we hypothesized that CD36 might regulate the in vivo senescence secretory program, impairing regeneration.

All three Sen cell types had higher CD36 protein expression in injured muscles **(****Figure 7C**). Cd36 expression was also induced in cells rendered senescent in response to etoposide treatment *in vitro,* confirming that distinct senescence triggers induce Cd36 *in vivo* and *in vitro.* We next analyzed muscles from young or old mice that had been treated for 4 days (starting at 3 DPI) with two doses of an anti-CD36 neutralizing antibody, or a control IgA antibody. CD36 blockade did not trigger a reduction in senescent cell number **(****Figure 7D**) but did induce a reduction in several SASP components, revealed by an analysis of proteins secreted by Sen cells **(****Figure 7E**). Remarkably, many of the CD36- regulated proteins coincided with upregulated SASP genes in Sen SCs, FAPs and Mcps, such as those encoding chemokines, cytokines and complement factors, including Ccl2, Ccl3, Ccl4, Il 1b, Il6, Igfbp5 and Clqa **(****Figure 7F**). These results indicated that CD36 blockade acts as a senomorphic rather than a senolytic, principally affecting the inflammatory SASPs. Notably, CD36 blockade improved muscle regeneration in both young and old mice **(****Figure 7G**) while reducing inflammation and fibrosis **(****Figure 7G**). Furthermore, regenerating muscles from anti-CD36 antibody-treated old mice showed increased force **(****Figure 7H**). Improved muscle regeneration after CD36 neutralization was comparable to the improvement observed after senescent cell elimination (either pharmacological or genetic) **(****Figure 2A**). These results thus indicate that CD36 inactivation unleashes repressive inflammatory downstream effects in SCs within the regenerative niche. This is consistent with myogenesis repression upon inhibition of CCR2 signaling in SCs by the chemokines CCL2, CCL7, and CCL8 (Blanc et al., 2020).

We next silenced Cd36 in freshly sorted Sen cells using specific siRNA (si-Cd36); scrambled siRNA (si- Scramble) was used as a control. Upon transplantation to injured muscle, si-Scramble-Sen cells increased the number of Sen cells in the damaged area and delayed its regeneration, whereas engraftment of Cd36- silenced Sen cells had no negative effects **(****Figure 7I**). Consistent with this result, the SASPs produced by freshly sorted Sen cells reduced SC proliferation in ex vivo transwell assays (**Figure 6G**), but this effect was not observed when Cd36 was silenced in senescent cells before co-culturing with SCs (**Figure 7J**

## Claims

1. CD36 inhibitor for use in promoting muscle regeneration, and/or in the treatment of muscle degeneration.

2. CD36 inhibitor for use, according to claim 1, wherein the CD36 inhibitor is an anti-CD36 antibody, or any antigen-binding fragment thereof comprising: a single chain antibody, F(ab')2, Fab, scFab or scFv.

3. CD36 inhibitor for use, according to claim 2, wherein the anti-CD36 antibody is a humanized antibody or a human antibody.

4. CD36 inhibitor for use, according to claim 1, wherein the CD36 inhibitor is a siRNA, a shRNA, an iRNA, an antisense RNA or DNA, specifically targeting CD36.

5. CD36 inhibitor for use, according to any of the claims 1 to 4, in the treatment of age-associated diseases or of muscular dystrophy.

6. Pharmaceutical composition comprising CD36 inhibitors according to any of claims 1 to 5, and, optionally, pharmaceutically acceptable carriers or excipients, for use in promoting muscle regeneration, or for use in the treatment of muscle degeneration.

7. Pharmaceutical composition for use, according to claim 6, in the treatment of age-associated diseases or of muscular dystrophy.

8. *In vitro* method for screening candidate molecules for promoting muscle regeneration, or for treating muscle degeneration, which comprises: a) assessing the level of expression of the CD36 after administering a candidate molecule and b) wherein, if after administering the candidate molecule, a statistically significant inhibition of CD36 expression is observed, with respect to a pre-established threshold expression level, this is indicative that the candidate molecule is effective in promoting muscle regeneration, or in the treatment of muscle degeneration.

9. *In vitro* method, according to claim 8, for screening candidate molecules for the treatment of age-associated diseases and/or muscular dystrophy.

10. *In vitro* method for monitoring the response of a patient to a treatment for promoting muscle regeneration, or for treating muscle degeneration, which comprises: a) assessing the level of expression of the CD36 after administering treatment and b) wherein, if after administering the treatment, a statistically significant inhibition of CD36 expression is observed, with respect to a pre-established threshold expression level, this is indicative that the patient is responding to the treatment.

11. *In vitro* method, according to claim 10, for monitoring the response of a patient to a treatment of age-associated diseases and/or muscular dystrophy.

12. *In vitro* use of CD36 for screening candidate molecules for promoting muscle regeneration, or for the treatment of muscle degeneration.

13. *In vitro* use of CD36, according to claim 12, for screening candidate molecules for treating age-associated diseases and/or muscular dystrophy.

14. *In vitro* use of CD36 for monitoring the response of a patient to a treatment for improving muscle regeneration, or for treating muscle degeneration.

15. *In vitro* use of CD36, according to claim 14, for monitoring the response of a patient to a treatment against age-associated diseases and/or muscular dystrophy.
